# EUROPEAN PATENT APPLICATION

(11) **EP 3 995 592 A1**
(43) Date of publication of application: **11.05.2022**
(21) Application number: 20206553.8
(22) Date of filing: 09.11.2020
(51) Int. Cl.: C12Q 1/6827, C12Q 1/6844

(54) **MULTIMERIC ISOTHERMAL NUCLEIC ACID AMPLIFICATION METHODS FOR POINT-OF-NEED DIAGNOSIS**

(71) Applicant: midge medical GmbH, 12103 Berlin (DE)
(72) Inventor: Riester, Markus, Wiesbaden (DE); Weidmann, Manfred, Berlin (DE)
(74) Representative: Eisenführ Speiser

(57) **Abstract**

The present invention relates to a multiplexing or multimeric method for fast isothermal amplification of nucleic acids, including DNA and RNA. Particularly, the invention relates to diagnostic methods for rapidly diagnosing, for example, at least two infectious agents, or at least two different targets in the same infectious agent, in a biological probe of interest. The invention further relates to a handheld and portable diagnostic system for performing the amplification method in a laboratory as well as in a non-laboratory environment. Further provided are suitable enzyme sequences, kits and uses of the method and the system.

SEQUENCE LISTING

<110>  midge medical GmbH

<120>  Multimeric isothermal nucleic acid amplification methods for
       point-of-need diagnosis

<130>  MB 1603-01EP

<160>  20

<170>  PatentIn version 3.5

<210>  1
<211>  358
<212>  PRT
<213>  Deinococcus geothermalis

<400>  1

Met Ser Lys Asp Asn Pro Lys Asp Phe Gly Thr Pro Ser Asp Ser Lys
1               5                   10                  15

Glu Arg Leu Lys Ala Ile Glu Thr Ala Met Thr Gln Ile Glu Lys Ala
            20                  25                  30

Phe Gly Lys Gly Ser Ile Met Arg Leu Gly Ala Glu Ser Lys Leu Asp
        35                  40                  45

Val Gln Ala Val Ser Thr Gly Ser Leu Ser Leu Asp Leu Ala Leu Gly
    50                  55                  60

Val Gly Gly Ile Pro Arg Gly Arg Ile Thr Glu Ile Tyr Gly Pro Glu
65                  70                  75                  80

Ser Gly Gly Lys Thr Thr Leu Ala Leu Ser Val Ile Ala Gln Ala Gln
                85                  90                  95

Arg Ala Gly Gly Thr Cys Ala Phe Ile Asp Ala Glu His Ala Leu Asp
            100                 105                 110

Pro Val Tyr Ala Arg Ser Leu Gly Val Asn Thr Asp Glu Leu Leu Val
        115                 120                 125

Ser Gln Pro Asp Asn Gly Glu Gln Ala Leu Glu Ile Met Glu Leu Leu
    130                 135                 140

Val Arg Ser Gly Ala Ile Asp Val Val Val Val Asp Ser Val Ala Ala
145                 150                 155                 160

Leu Thr Pro Arg Ala Glu Ile Glu Gly Glu Met Gly Asp Ser Leu Pro
                165                 170                 175

Gly Leu Gln Ala Arg Leu Met Ser Gln Ala Leu Arg Lys Leu Thr Ala
            180                 185                 190

Ile Leu Ser Lys Thr Gly Thr Ala Ala Ile Phe Ile Asn Gln Val Arg
        195                 200                 205

Glu Lys Ile Gly Val Met Tyr Gly Asn Pro Glu Thr Thr Thr Gly Gly
    210                 215                 220

Arg Ala Leu Lys Phe Tyr Ala Ser Val Arg Leu Asp Val Arg Lys Ile
225                 230                 235                 240

Gly Gln Pro Val Lys Leu Gly Asn Asp Ala Val Gly Asn Thr Val Lys
                245                 250                 255

Val Lys Thr Val Lys Asn Lys Val Ala Pro Pro Phe Lys Glu Val Glu
            260                 265                 270

Leu Thr Leu Leu Tyr Gly Lys Gly Phe Asp Gln Leu Ser Asp Leu Val
        275                 280                 285

Thr Leu Ala Ala Asp Met Asp Ile Ile Lys Lys Ala Gly Ser Phe Tyr
    290                 295                 300

Ser Tyr Gly Glu Glu Arg Ile Gly Gln Gly Lys Glu Lys Ala Ile Ala
305                 310                 315                 320

Tyr Ile Ala Glu Arg Pro Glu Leu Glu Gln Glu Ile Arg Asp Arg Val
                325                 330                 335

Leu Ala Ala Ile Lys Glu Gly Arg Asp Pro Ile Ala Ala Val Pro Glu
            340                 345                 350

Thr Pro Ala Leu Ala Glu
        355

<210> 2
<211> 222
<212> PRT
<213> Deinococcus geothermalis

<400> 2

Met Lys Tyr Pro Pro Ser Leu Val Gly Leu Ile Arg Glu Leu Ser Arg
1               5                   10                  15

Leu Pro Gly Ile Gly Pro Lys Ser Ala Gln Arg Leu Ala Phe Tyr Leu
            20                  25                  30

Phe Glu Gln Pro Arg Glu Asp Ile Glu Arg Leu Ala Gly Ala Ile Leu
        35                  40                  45

Glu Ala Lys Arg Asp Leu His Thr Cys Pro Val Cys Phe Asn Ile Thr
    50                  55                  60

Asp Ala Glu Arg Cys Asp Val Cys Ser Asp Pro Thr Arg Asp Gln Ser
65                  70                  75                  80

Val Ile Cys Val Val Glu Glu Pro Gly Asp Val Ile Ala Ile Glu Arg
                85                  90                  95

Ser Gly Glu Tyr Arg Gly Leu Tyr His Val Leu His Gly Ala Leu Ser
            100                 105                 110

Pro Met Asn Gly Val Gly Pro Asp Arg Leu Gln Ile Arg Pro Leu Leu
        115                 120                 125

Pro Arg Val Gln Asp Gly Met Glu Val Ile Leu Ala Thr Gly Thr Thr
    130                 135                 140

Val Glu Gly Asp Ala Thr Ala Leu Tyr Leu Gln Arg Leu Leu Glu Pro
145                 150                 155                 160

Leu Gly Ala Val Val Ser Arg Ile Ala Tyr Gly Leu Pro Val Gly Gly
                165                 170                 175

Ala Leu Glu Tyr Ala Asp Glu Val Thr Leu Gly Arg Ala Leu Ser Gly
            180                 185                 190

Arg Arg Arg Val Ser Glu Pro Ala Ser Pro Pro Pro Pro Arg Arg Asn
        195                 200                 205

Asp Glu Glu Gln Asp Gly Ala Pro Ala Arg Pro Pro Ser His
    210                 215                 220

<210>  3
<211>  243
<212>  PRT
<213>  Deinococcus geothermalis

<400>  3

Met Arg Ser Arg Ser Ala Asn Arg Ser Gly Ile Val Ile Arg Arg Arg
1               5                   10                  15

Val Thr Pro Ala Gly Asp Ile Ile Val Thr Leu Leu Thr Pro Gln Gly
            20                  25                  30

Lys Val Lys Ala Ile Ala Arg Gly Gly Val Arg Gly Ala Leu Ser Ser
        35                  40                  45

Arg Leu Asn Leu Phe His His Val Ala Ile Gln Leu Tyr Gln Thr Pro
    50                  55                  60

Gln Ala Asp Leu Ala Thr Val Gln Gln Thr Val Leu Glu Gly Ala Leu
65                  70                  75                  80

Pro Lys Leu Ala Glu Pro Glu Arg Tyr Ala Phe Ala His Leu Met Ala
                85                  90                  95

Glu Leu Ala Asp Ala Leu Phe Gln Glu Gly Glu Phe Ser Glu Gln Ala
            100                 105                 110

Phe Glu Leu Phe Ala Gly Ala Leu Arg Gly Ile Ser His Gln Pro Asp
        115                 120                 125

Pro Glu Trp Val Ala Leu Val Met Ser Tyr Lys Leu Leu Gly Leu Ala
    130                 135                 140

Gly Phe Val Pro Gln Thr Gly Arg Cys Ala Arg Cys Gly Ala Ala Ala
145                 150                 155                 160

Pro Thr His Pro Asp Pro Leu Gly Gly Gln Leu Leu Cys Gly Ala Cys
                165                 170                 175

Ala Ser Leu Pro Ala Tyr Pro Pro Glu Gly Leu Asp Phe Leu Arg Asn
            180                 185                 190

Val Val Arg Arg Ser Val Arg Ala Asn Met Asp Arg Pro Val Pro Glu
        195                 200                 205

Glu Gln Arg Pro Ala Leu Trp Arg Ala Leu Glu Arg Phe Val Thr Val
    210                 215                 220

Gln Val Gly Asn Val Gln Ser Trp Arg Gln Leu Val Pro Ala Gly Ala
225                 230                 235                 240

Val Ser Val

<210>  4
<211>  178
<212>  PRT
<213>  Deinococcus geothermalis

<400>  4

Met Leu His Ile Glu Phe Ile Thr Asp Leu Gly Ala Lys Val Thr Val
1               5                   10                  15

Asp Val Glu Ser Ala Asp Lys Leu Leu Asp Val Gln Arg Gln Tyr Gly
            20                  25                  30

Arg Leu Gly Trp Thr Ser Gly Glu Val Pro Val Gly Gly Tyr Gln Phe
        35                  40                  45

Pro Leu Glu Asn Glu Pro Asp Phe Asp Trp Ser Leu Ile Gly Ala Arg
    50                  55                  60

Lys Trp Thr Asn Pro Glu Gly Glu Glu Met Ile Leu His Arg Gly His
65                  70                  75                  80

Ala Tyr Arg Arg Arg Glu Leu Glu Ala Val Asp Ser Arg Lys Met Lys
                85                  90                  95

Leu Pro Ala Ala Val Lys Tyr Ser Arg Gly Ala Lys Asn Thr Asp Pro
            100                 105                 110

Glu His Val Arg Glu Lys Ala Asp Gly Glu Phe Glu Tyr Val Thr Leu
        115                 120                 125

Ala Ile Phe Arg Gly Gly Lys Arg Gln Glu Arg Tyr Ala Val Pro Gly
    130                 135                 140

Ser Asn Arg Pro Gln Ala Gly Ala Pro Ala Arg Ser Ala Ala Thr Arg
145                 150                 155                 160

Ala Gln Gly Ala Arg Pro Gly Ala Val Ala Val Gln Asp Glu Glu Thr
                165                 170                 175

Pro Phe

<210>  5
<211>  876
<212>  PRT
<213>  Geobacillus stearothermophilus

<400>  5

Met Lys Asn Lys Leu Val Leu Ile Asp Gly Asn Ser Val Ala Tyr Arg
1               5                   10                  15

Ala Phe Phe Ala Leu Pro Leu Leu His Asn Asp Lys Gly Ile His Thr
            20                  25                  30

Asn Ala Val Tyr Gly Phe Thr Met Met Leu Asn Lys Ile Leu Ala Glu
        35                  40                  45

Glu Gln Pro Thr His Ile Leu Val Ala Phe Asp Ala Gly Lys Thr Thr
    50                  55                  60

Phe Arg His Glu Thr Phe Gln Asp Tyr Lys Gly Gly Arg Gln Gln Thr
65                  70                  75                  80

Pro Pro Glu Leu Ser Glu Gln Phe Pro Leu Leu Arg Glu Leu Leu Lys
                85                  90                  95

Ala Tyr Arg Ile Pro Ala Tyr Glu Leu Asp His Tyr Glu Ala Asp Asp
            100                 105                 110

Ile Ile Gly Thr Met Ala Ala Arg Ala Glu Arg Glu Gly Phe Ala Val
        115                 120                 125

Lys Val Ile Ser Gly Asp Arg Asp Leu Thr Gln Leu Ala Ser Pro Gln
    130                 135                 140

Val Thr Val Glu Ile Thr Lys Lys Gly Ile Thr Asp Ile Glu Ser Tyr
145                 150                 155                 160

Thr Pro Glu Thr Val Val Glu Lys Tyr Gly Leu Thr Pro Glu Gln Ile
                165                 170                 175

Val Asp Leu Lys Gly Leu Met Gly Asp Lys Ser Asp Asn Ile Pro Gly
            180                 185                 190

Val Pro Gly Ile Gly Glu Lys Thr Ala Val Lys Leu Leu Lys Gln Phe
        195                 200                 205

Gly Thr Val Glu Asn Val Leu Ala Ser Ile Asp Glu Ile Lys Gly Glu
    210                 215                 220

Lys Leu Lys Glu Asn Leu Arg Gln Tyr Arg Asp Leu Ala Leu Leu Ser
225                 230                 235                 240

Lys Gln Leu Ala Ala Ile Cys Arg Asp Ala Pro Val Glu Leu Thr Leu
                245                 250                 255

Asp Asp Ile Val Tyr Lys Gly Glu Asp Arg Glu Lys Val Val Ala Leu
            260                 265                 270

Phe Gln Glu Leu Gly Phe Gln Ser Phe Leu Asp Lys Met Ala Val Gln
        275                 280                 285

Thr Asp Glu Gly Glu Lys Pro Leu Ala Gly Met Asp Phe Ala Ile Ala
    290                 295                 300

Asp Ser Val Thr Asp Glu Met Leu Ala Asp Lys Ala Ala Leu Val Val
305                 310                 315                 320

Glu Val Val Gly Asp Asn Tyr His His Ala Pro Ile Val Gly Ile Ala
                325                 330                 335

Leu Ala Asn Glu Arg Gly Arg Phe Phe Leu Arg Pro Glu Thr Ala Leu
            340                 345                 350

Ala Asp Pro Lys Phe Leu Ala Trp Leu Gly Asp Glu Thr Lys Lys Lys
        355                 360                 365

Thr Met Phe Asp Ser Lys Arg Ala Ala Val Ala Leu Lys Trp Lys Gly
    370                 375                 380

Ile Glu Leu Arg Gly Val Val Phe Asp Leu Leu Leu Ala Ala Tyr Leu
385                 390                 395                 400

Leu Asp Pro Ala Gln Ala Ala Gly Asp Val Ala Ala Val Ala Lys Met
                405                 410                 415

His Gln Tyr Glu Ala Val Arg Ser Asp Glu Ala Val Tyr Gly Lys Gly
            420                 425                 430

Ala Lys Arg Thr Val Pro Asp Glu Pro Thr Leu Ala Glu His Leu Ala
        435                 440                 445

Arg Lys Ala Ala Ala Ile Trp Ala Leu Glu Glu Pro Leu Met Asp Glu
    450                 455                 460

Leu Arg Arg Asn Glu Gln Asp Arg Leu Leu Thr Glu Leu Glu Gln Pro
465                 470                 475                 480

Leu Ala Gly Ile Leu Ala Asn Met Glu Phe Thr Gly Val Lys Val Asp
                485                 490                 495

Thr Lys Arg Leu Glu Gln Met Gly Ala Glu Leu Thr Glu Gln Leu Gln
            500                 505                 510

Ala Val Glu Arg Arg Ile Tyr Glu Leu Ala Gly Gln Glu Phe Asn Ile
        515                 520                 525

Asn Ser Pro Lys Gln Leu Gly Thr Val Leu Phe Asp Lys Leu Gln Leu

530                 535                 540

Pro Val Leu Lys Lys Thr Lys Thr Gly Tyr Ser Thr Ser Ala Asp Val
545                 550                 555                 560

Leu Glu Lys Leu Ala Pro His His Glu Ile Val Glu His Ile Leu His
                565                 570                 575

Tyr Arg Gln Leu Gly Lys Leu Gln Ser Thr Tyr Ile Glu Gly Leu Leu
            580                 585                 590

Lys Val Val His Pro Val Thr Gly Lys Val His Thr Met Phe Asn Gln
        595                 600                 605

Ala Leu Thr Gln Thr Gly Arg Leu Ser Ser Val Glu Pro Asn Leu Gln
    610                 615                 620

Asn Ile Pro Ile Arg Leu Glu Glu Gly Arg Lys Ile Arg Gln Ala Phe
625                 630                 635                 640

Val Pro Ser Glu Pro Asp Trp Leu Ile Phe Ala Ala Asp Tyr Ser Gln
                645                 650                 655

Ile Glu Leu Arg Val Leu Ala His Ile Ala Glu Asp Asp Asn Leu Ile
            660                 665                 670

Glu Ala Phe Arg Arg Gly Leu Asp Ile His Thr Lys Thr Ala Met Asp
        675                 680                 685

Ile Phe His Val Ser Glu Glu Asp Val Thr Ala Asn Met Arg Arg Gln
    690                 695                 700

Ala Lys Ala Val Asn Phe Gly Ile Val Tyr Gly Ile Ser Asp Tyr Gly
705                 710                 715                 720

Leu Ala Gln Asn Leu Asn Ile Thr Arg Lys Glu Ala Ala Glu Phe Ile
                725                 730                 735

Glu Arg Tyr Phe Ala Ser Phe Pro Gly Val Lys Gln Tyr Met Asp Asn
            740                 745                 750

Ile Val Gln Glu Ala Lys Gln Lys Gly Tyr Val Thr Thr Leu Leu His
        755                 760                 765

Arg Arg Arg Tyr Leu Pro Asp Ile Thr Ser Arg Asn Phe Asn Val Arg
    770                 775                 780

Ser Phe Ala Glu Arg Thr Ala Met Asn Thr Pro Ile Gln Gly Ser Ala
785                 790                 795                 800

Ala Asp Ile Ile Lys Lys Ala Met Ile Asp Leu Ser Val Arg Leu Arg
                805                 810                 815

Glu Glu Arg Leu Gln Ala Arg Leu Leu Leu Gln Val His Asp Glu Leu
            820                 825                 830

Ile Leu Glu Ala Pro Lys Glu Glu Ile Glu Arg Leu Cys Arg Leu Val
        835                 840                 845

Pro Glu Val Met Glu Gln Ala Val Ala Leu Arg Val Pro Leu Lys Val
    850                 855                 860

Asp Tyr His Tyr Gly Pro Thr Trp Tyr Asp Ala Lys
865                 870                 875

<210>  6
<211>  560
<212>  PRT
<213>  Human immunodeficiency virus type 1

<400>  6

Pro Ile Ser Pro Ile Ala Pro Val Pro Val Lys Leu Lys Pro Gly Met
1               5                   10                  15

Asp Gly Pro Lys Val Lys Gln Trp Pro Leu Ser Lys Glu Lys Ile Glu
            20                  25                  30

Ala Leu Thr Ala Ile Cys Gln Glu Met Glu Gln Glu Gly Lys Ile Ser
        35                  40                  45

Arg Ile Gly Pro Glu Asn Pro Tyr Asn Thr Pro Ile Phe Ala Ile Lys
    50                  55                  60

Lys Lys Asp Ser Thr Lys Trp Arg Lys Leu Val Asp Phe Arg Glu Leu
65                  70                  75                  80

Asn Lys Arg Thr Gln Asp Phe Trp Glu Val Gln Leu Gly Ile Pro His
                85                  90                  95

Pro Gly Gly Leu Lys Gln Lys Arg Ser Val Thr Val Leu Asp Val Gly
            100                 105                 110

Asp Ala Tyr Phe Ser Cys Pro Leu Asp Pro Asp Phe Arg Lys Tyr Thr
        115                 120                 125

Ala Phe Thr Ile Pro Ser Val Asn Asn Glu Thr Pro Gly Val Arg Tyr
    130                 135                 140

Gln Tyr Asn Val Leu Pro Gln Gly Trp Lys Gly Ser Pro Ala Ile Phe
145                 150                 155                 160

Gln Ser Ser Met Thr Lys Ile Leu Asp Pro Phe Arg Lys Asp Asn Pro
                165                 170                 175

Glu Leu Glu Ile Cys Gln Tyr Met Asp Asp Leu Tyr Val Gly Ser Asp
            180                 185                 190

Leu Pro Leu Thr Glu His Arg Lys Arg Val Glu Ser Leu Arg Glu His
        195                 200                 205

Leu Tyr Gln Trp Gly Phe Thr Thr Pro Asp Lys Lys His Gln Lys Glu
    210                 215                 220

Pro Pro Phe Leu Trp Met Gly Tyr Glu Leu His Pro Asp Lys Trp Thr
225                 230                 235                 240

Val Gln Pro Ile Lys Leu Pro Asn Lys Asp Val Trp Thr Val Asn Asp
                245                 250                 255

Ile Gln Lys Leu Ile Gly Lys Leu Asn Trp Ala Ser Gln Ile Tyr Gln
            260                 265                 270

Gly Ile Arg Ile Arg Glu Leu Cys Lys Leu Ile Arg Gly Thr Lys Ser
        275                 280                 285

Leu Thr Glu Val Val Pro Leu Ser Lys Glu Ala Glu Met Glu Leu Glu
    290                 295                 300

Glu Asn Arg Glu Lys Leu Lys Glu Pro Val His Gly Val Tyr Tyr Gln
305                 310                 315                 320

Pro Asp Lys Asp Leu Trp Val Asn Ile Gln Lys Gln Gly Glu Gly Gln
                325                 330                 335

Trp Thr Tyr Gln Ile Tyr Gln Asp Glu His Lys Asp Leu Lys Thr Gly
            340                 345                 350

Lys Tyr Thr Arg Gln Lys Ala Ser His Thr Asn Asp Ile Arg Gln Leu
        355                 360                 365

Ala Glu Val Leu Gln Lys Val Ser Gln Glu Ser Ile Val Ile Trp Gly
    370                 375                 380

Lys Leu Pro Lys Phe Lys Leu Pro Val Thr Arg Glu Thr Trp Glu Thr
385                 390                 395                 400

Trp Trp Ala Asp Tyr Trp Gln Ala Thr Trp Ile Pro Glu Trp Glu Tyr
                405                 410                 415

Val Ser Thr Pro Pro Leu Ile Lys Leu Trp Tyr Arg Leu Glu Ser Glu
            420                 425                 430

Pro Ile Met Gly Ala Glu Thr Tyr Tyr Val Asp Gly Ala Ala Asn Arg
        435                 440                 445

Asp Thr Lys Leu Gly Lys Ala Gly Tyr Val Thr Glu Gln Gly Lys Gln
    450                 455                 460

Lys Ile Ile Lys Leu Asn Glu Thr Thr Asn Gln Lys Ala Glu Leu Met
465                 470                 475                 480

Ala Val Leu Leu Ala Leu Gln Asp Ser Lys Glu Lys Val Asn Ile Val
                485                 490                 495

Thr Asp Ser Gln Tyr Val Leu Gly Ile Ile Ser Ser Gln Pro Thr Gln
            500                 505                 510

Ser Glu Ser Pro Ile Val Gln Gln Ile Ile Glu Glu Leu Thr Lys Lys
        515                 520                 525

Glu Gln Val Tyr Leu Thr Trp Val Pro Ala His Lys Gly Ile Gly Gly
    530                 535                 540

Asn Glu Lys Ile Asp Lys Leu Val Ser Lys Asp Ile Arg Arg Val Leu
545                 550                 555                 560

<210>  7
<211>  726
<212>  PRT
<213>  Deinococcus geothermalis

<400>  7

Met Leu Ala Pro His His Ala Phe Ser Cys Glu Val Pro Thr Val Pro
1               5                   10                  15

Ala Glu Pro Ser Ile Ser Pro Ala Pro Glu Val Ala Ala Pro Ala Thr
            20                  25                  30

Gln Gly Thr Arg Arg Lys Lys Ala Ala Arg Thr Ala Ala Leu Pro Ala
        35                  40                  45

Glu Gly Thr Arg Thr Phe Ser Thr Val Ala Asn Pro Asp Ser Ala Phe
    50                  55                  60

Leu Asn Arg Glu Leu Ser Trp Leu Ala Phe Asn Ser Arg Val Leu Ala
65                  70                  75                  80

Glu Ala Gln Asp Pro Arg Asn Pro Pro Leu Glu Arg Leu Lys Tyr Ala
                85                  90                  95

Ala Ile Cys Gly Ser Asn Leu Asp Glu Phe Phe Met Val Arg Val Ala
            100                 105                 110

Gly Ile His Arg Gln Ile Ala Ala Gly Val Asn Thr Pro Gly Pro Asp
        115                 120                 125

Gly Leu Leu Pro Arg Glu Thr Leu Ala Leu Val Arg Glu Arg Thr His
    130                 135                 140

Ile Met Leu Arg Gln Ile Glu Lys Ala Ala Arg Arg Asn Leu Lys Asp
145                 150                 155                 160

Leu Val Ala Gln Gly Val Arg Leu Val Arg Val Ala Asp Leu Gly Lys
                165                 170                 175

Arg Ala Arg Ala Ala Leu Arg Glu His Tyr Leu Ser Glu Ile Gln Pro
            180                 185                 190

Val Leu Thr Pro Leu Val Val Asp Pro Ser His Pro Phe Pro Tyr Leu
        195                 200                 205

Ser Asn Leu Ser Leu Asn Leu Ala Val Leu Leu Asp Gly Gly Glu Gly
    210                 215                 220

Glu Asp Pro Glu Phe Ala Arg Val Lys Val Pro Val Gly Val Leu Pro
225                 230                 235                 240

Arg Val Val Lys Val Gly Asp His Leu Leu Leu Leu Glu Asp Val Ile
                245                 250                 255

Ala Ala His Leu Asp Asp Leu Phe Lys Gly Arg Arg Val Leu Ala Ala
            260                 265                 270

His Thr Phe Arg Val Thr Arg Asn Thr Asp Tyr Glu Phe Glu Glu Glu
        275                 280                 285

Glu Ala Glu Asp Leu Leu Ala Thr Ile Glu Asp Gly Leu Arg Arg Arg
    290                 295                 300

Arg Phe Gly Ser Ala Val Arg Leu Glu Val Val Gln Asp Thr Pro Pro
305                 310                 315                 320

Arg Ile Ile Thr Phe Leu Gln Glu Arg Leu Arg Leu Ala Ala Glu Asp
                325                 330                 335

Val Phe Leu Leu Glu Gly Pro Leu Gly Thr Ala Asp Leu Met Ala Leu
            340                 345                 350

Pro Val Asp Arg Pro Asp Leu Ser Phe Pro Pro Tyr Val Pro Ala Val
        355                 360                 365

Pro Asp Leu Asp Gly Asp Glu Glu Asn Gly Ile Phe Asp Thr Leu Arg
    370                 375                 380

Gln Gly Asp Val Leu Leu His His Pro Tyr Asp Gly Phe Thr Asn Val
385                 390                 395                 400

Leu Asn Phe Leu Glu Glu Ala Ala Arg Asp Pro Gln Val Leu Ala Ile
                405                 410                 415

Lys Gln Thr Leu Tyr Arg Thr Gly Asp Asp Pro Arg Leu Leu Ser Ala
            420                 425                 430

Leu Arg Thr Ala Ala Glu Asn Gly Lys Gln Val Val Ala Leu Ile Glu
        435                 440                 445

Leu Lys Ala Arg Phe Asp Glu Gln Arg Asn Ile Ser Trp Ala Arg Lys
    450                 455                 460

Leu Glu Arg Ala Gly Ala His Val Val Tyr Gly Met Ala Gly Leu Lys
465                 470                 475                 480

Thr His Ala Lys Val Thr Leu Val Val Arg Arg Glu Glu Gly Gly Leu
                485                 490                 495

Arg Arg Tyr Val His Ile Gly Thr Gly Asn Tyr Asn Pro Lys Thr Ala
            500                 505                 510

Arg Leu Tyr Thr Asp Leu Ser Leu Leu Ser Ala Asp Pro Asp Leu Gly
        515                 520                 525

Ala Asp Val Ser Glu Leu Phe Asn His Leu Thr Gly Tyr Ala Glu Ala
    530                 535                 540

Thr Tyr Thr Arg Leu Leu Val Ala Pro Asp Thr Ala Arg Ala Asp Leu

545                 550                 555                 560

Glu Ala Leu Leu Asp Arg Glu Ala Gln His Ala Arg Thr Gly Gln Glu
                565                 570                 575

Ala Trp Val Arg Val Lys Val Asn Gln Leu Thr Asp Pro Gly Met Ile
            580                 585                 590

Glu Ala Leu Tyr Arg Ala Ser Gln Ala Gly Val Arg Val Glu Leu Ile
        595                 600                 605

Ile Arg Gly Val Cys Cys Leu Arg Pro Gly Val Pro Asp Leu Ser Glu
    610                 615                 620

Thr Val Arg Val Arg Ser Leu Leu Gly Arg Tyr Leu Glu His Ala Arg
625                 630                 635                 640

Ile Tyr Ala Phe Ala Asn Ala Gly Ser Pro Glu Val Tyr Phe Gly Ser
                645                 650                 655

Ala Asp Trp Met Ser Arg Asn Leu Asp Arg Arg Val Glu Val Ile Ala
            660                 665                 670

Pro Val Leu Asp Asp Arg His Arg Asp Glu Leu Leu Arg Leu Leu Ala
        675                 680                 685

Thr Glu Trp Ala Asp Glu Arg Gly Ser Trp Glu Leu His Ala Asp Gly
    690                 695                 700

Ile Tyr Gln Lys Leu Pro Gly Asp Phe Ser Ala Gln Gln Ala Phe Ala
705                 710                 715                 720

Glu Ala Arg His Pro Gly
                725

<210>  8
<211>  257
<212>  PRT
<213>  Archaeoglobus fulgidus

<400>  8

Met Leu Lys Ile Ala Thr Phe Asn Val Asn Ser Ile Arg Ser Arg Leu
1               5                   10                  15

His Ile Val Ile Pro Trp Leu Lys Glu Asn Lys Pro Asp Ile Leu Cys
            20                  25                  30

Met Gln Glu Thr Lys Val Glu Asn Arg Lys Phe Pro Glu Ala Asp Phe

35                  40                  45

His Arg Ile Gly Tyr His Val Val Phe Ser Gly Ser Lys Gly Arg Asn
    50                  55                  60

Gly Val Ala Ile Ala Ser Leu Glu Glu Pro Glu Asp Val Ser Phe Gly
65                  70                  75                  80

Leu Asp Ser Glu Pro Lys Asp Glu Asp Arg Leu Ile Arg Ala Lys Ile
                85                  90                  95

Ala Gly Ile Asp Val Ile Asn Thr Tyr Val Pro Gln Gly Phe Lys Ile
            100                 105                 110

Asp Ser Glu Lys Tyr Gln Tyr Lys Leu Gln Trp Leu Glu Arg Leu Tyr
        115                 120                 125

His Tyr Leu Gln Lys Thr Val Asp Phe Arg Ser Phe Ala Val Trp Cys
    130                 135                 140

Gly Asp Met Asn Val Ala Pro Glu Pro Ile Asp Val His Ser Pro Asp
145                 150                 155                 160

Lys Leu Lys Asn His Val Cys Phe His Glu Asp Ala Arg Arg Ala Tyr
                165                 170                 175

Lys Lys Ile Leu Glu Leu Gly Phe Val Asp Val Leu Arg Lys Ile His
            180                 185                 190

Pro Asn Glu Arg Ile Tyr Thr Phe Tyr Asp Tyr Arg Val Lys Gly Ala
        195                 200                 205

Ile Glu Arg Gly Leu Gly Trp Arg Val Asp Ala Ile Leu Ala Thr Pro
    210                 215                 220

Pro Leu Ala Glu Arg Cys Val Asp Cys Tyr Ala Asp Ile Lys Pro Arg
225                 230                 235                 240

Leu Ala Glu Lys Pro Ser Asp His Leu Pro Leu Val Ala Val Phe Asp
                245                 250                 255

Val

<210>  9
<211>  32
<212>  DNA
<213>  Artificial Sequence

<220>
<223>  Forward Primer

<400>  9
tatgccatta gtgcaaagaa tagagctcgc ac                                    32


<210>  10
<211>  32
<212>  DNA
<213>  Artificial Sequence

<220>
<223>  Reverse Primer

<400>  10
caaccaccat agaatttgct tgttccaatt ac                                    32


<210>  11
<211>  52
<212>  DNA
<213>  Artificial Sequence

<220>
<223>  Exo-Probe


<220>
<221>  misc_feature
<222>  (31)..(33)
<223>  n31= BHQ1-dt (bT), n32=Tetrahydrofuran (X), n33=Fam-dT (fT)

<400>  11
tcctctagtg gcggctattg atttcaataa nnnttgatga aactgtctat tg              52


<210>  12
<211>  33
<212>  DNA
<213>  Artificial Sequence

<220>
<223>  Forward Primer

<400>  12
gaagagacag gtacgttaat agttaatagc gta                                   33


<210>  13
<211>  32
<212>  DNA
<213>  Artificial Sequence

<220>
<223>  Reverse Primer

<400>  13
aaaaagaagg ttttacaaga ctcacgttaa ca                                    32


<210>  14

<211>  44
<212>  DNA
<213>  Artificial Sequence

<220>
<223>  Exo-Probe


<220>
<221>  misc_feature
<222>  (26)..(28)
<223>  n26= BHQ1-dt (bT), n27=Tetrahydrofuran (X), n28=Fam-dT (fT)

<400>  14
atcgaagcgc agtaaggatg gctagnnnta actagcaaga atac                    44


<210>  15
<211>  32
<212>  DNA
<213>  Artificial Sequence

<220>
<223>  Forward Primer

<400>  15
cctcttctcg ttcctcatca cgtagtcgca ac                                 32


<210>  16
<211>  32
<212>  DNA
<213>  Artificial Sequence

<220>
<223>  Reverse Primer

<400>  16
agtgacagtt tggccttgtt gttgttggcc tt                                 32


<210>  17
<211>  50
<212>  DNA
<213>  Artificial Sequence

<220>
<223>  Exo-Probe


<220>
<221>  misc_feature
<222>  (31)..(33)
<223>  n31= BHQ1-dt (bT), n32=Tetrahydrofuran (X), n33=Fam-dT (fT)

<400>  17
tagaatggct ggcaatggcg gtgatgctgc nnnttgcttt gctgctgctt              50


<210>  18
<211>  32
<212>  DNA
<213>  Artificial Sequence

<220>
<223>  Forward Primer

<400>  18
aacatgttgt gccaaccacc atagaatttg ct                                    32


<210>  19
<211>  32
<212>  DNA
<213>  Artificial Sequence

<220>
<223>  Reverse Primer

<400>  19
gtgcgagctc tattctttgc actaatggca ta                                    32


<210>  20
<211>  52
<212>  DNA
<213>  Artificial Sequence

<220>
<223>  Mimic Probe

<400>  20
aaattctcta ttgagcataa gaatattgct gcggttacat tcttgtttgc cg              52

## Description

### Technical Field

The present invention relates to a multiplexing or multimeric method for fast isothermal amplification of nucleic acids, including DNA and RNA. Particularly, the invention relates to diagnostic methods for rapidly diagnosing, for example, at least two infectious agents, or at least two different targets in the same infectious agent, in a biological sample of interest. The invention further relates to a handheld and portable diagnostic system for performing the amplification method in a laboratory as well as in a non-laboratory environment. Further provided are suitable enzyme sequences, kits and uses of the method and the system.

### Background of the Invention

The SARS-CoV-2 virus outbreak declared as a world pandemic by the WHO in March 2020 quite strikingly demonstrated the need for reliable and fast diagnostics, particularly for newly emerged infectious diseases for which no prophylactic vaccine or curative medicine is available. Hitherto, sensitive and fast diagnostic tools reliably identifying the SARS-CoV-2 virus in a short time, are not yet available. This is highly problematic as there is a significant time gap between testing and the availability of the test result so that an isolation of infected individuals can often only be initiated after a further virus spread could take place. Still, this is only one scenario strikingly illustrating that testing and diagnosis for the presence of pathogens of various kinds (viruses, bacteria, fungi, or parasites) presently takes too much time until a reliable result is available. For example, infections caused by multidrug resistant bacteria represent a therapeutic challenge both in clinical settings and in livestock production, returnees from private and business trips may wish to receive an early diagnosis of potential infections before spreading a potential infection to the social environment after return, pregnant women and/or individuals belonging to risk groups having a susceptible immune defense may wish to have early certainty on their health status etc.

All these scenarios have in common that usually the presence of more than one nucleic acid sequence of different infections agent and/or information regarding the presence of more than one target sequence is needed, and this information is needed urgently. In case of infectious agents, it is very important to isolate infected individuals as soon as possible and to provide a specific treatment regimen for the respective disease as soon as possible. But also in any kind of care area (clinical, retirement home), it is still a problem at date to test for the presence of certain infectious agents on or within a patient, or on a potentially contaminated surface in view of the fact that quick and reliable multiplexing tests are not easily available.

Nucleic acid amplification techniques (NAAT) like molecular real-time PCR assays are usually very sensitive, and specific but when it comes to time-to-result, PCR still has the inherent disadvantage to require highly-equipped laboratories and well-trained personnel. Therefore, new portable diagnostic solutions having a good specificity and sensitivity and being able to provide reliable results in situ at the place of testing are urgently needed.

Regarding nucleic acid-based preparative, cloning and diagnostic techniques, the development of polymerase chain reaction (PCR) in the 1980^{ies} by the later Nobel laureate Kary Mullis and team as rapid and reliable method to amplify DNA revolutionized molecular biology in general, as scientists suddenly had the opportunity to obtain millions of copies of a DNA target molecule of interest in short time. Simplicity and efficiency (e.g., nowadays possibilities to perform single-molecule/cell PCR) represent significant advantages of PCR techniques.

Still, PCR suffers from certain drawbacks, including the inherently necessary need for iterative rounds of thermal cycling and the shift between different temperatures (repeated cycles of two or three temperature-dependent steps during the amplification process) and the use of high (>90°C) temperatures. These drawbacks have led to the development of alternative amplification methods.

An important class of PCR alternatives are so called isothermal amplification methods (for a review, see Zanoli and Spoto, Biosensors (Basel), 2012 3(1): 18-43)). The huge advantage over PCR is the fact that isothermal nucleic acid amplification methods are not requiring any thermal cycling at all, but can be conducted at constant temperatures. This makes the amplification process much easier to operate and to control. Further, less energy is needed than for PCR methods, the latter inherently requiring rapid heating and cooling steps. The constant temperature of isothermal methods additionally allows fully enclosed micro-structured devices into which performing the isothermal amplification reduces the risk of sample contamination and implies low sample consumption, multiplex DNA analysis, integration and portable devices realization. Finally, the constant temperature would be highly preferably for point-of-need and/or portable diagnostic devices, as recently developed by the present applicant (DE 10 2020 109 744.1 which is incorporated herein by reference).

Isothermal amplification strategies available at date include nucleic acid sequence-based amplification (NASBA), loop-mediated isothermal amplification (LAMP), helicase-dependent amplification (HDA), rolling circle amplification (RCA), multiple displacement amplification (MDA), recombinase polymerase amplification (RPA) (see again: Zanoli and Spoto, 2012, *supra*; for a comprehensive survey on nowadays RPA techniques: Li et al., Analyst, 2019, 144, 31, pp. 31 to 67), or strand invasion based amplification (SIBA^{®}- SIBA in the following) (Hoser et al., PLoS ONE 9(11): e112656. doi:10.1371/journal.pone.0112656). Still, just as PCR isothermal technologies too, tend to produce non-specific amplification products as major drawbacks.

Whereas, for example, NASBA, RPA and HDA (and certain kinds of quantitative PCR) rely on the use of target-specific probes to provide for higher specificity of the reaction, LAMP uses additional primers instead of probes together with a strand displacement polymerase (Notomi et al., 2000, Nucleic Acids Research 28: e63) . The use of numerous large primers in a at least 30 min incubation, however, has been shown to produce false-positive results - an outcome definitely to be avoided during diagnostic applications, particularly for diagnosing infectious diseases. Further, there is the problem that LAMP reactions require a high temperature of 65°C, which is unfavourable for diagnostic devices for use in a home environment due to regulatory and practical issues. Consequently, LAMP will not be the method of choice in case a diagnostic nucleic acid amplification result is needed in a short time in a handheld device, which has to fulfill certain regulatory requirements to be suitable for non-laboratory use.

RPA and SIBA technology both rely on the use of a recombinase during the binding and amplification process. Initially, a nucleoprotein complex constituted by oligonucleotide primers and the recombinase proteins is formed for RPA- and SIBA-type nucleic acid amplification strategies, these complexes facilitating the primer binding to the template DNA. Due to their short run times (around 15 min), unspecific amplification has not been observed. A certain advantage is the fact the recombinase can tolerate at least one incorrect base without preventing strand invasion necessary to start the reaction. Overall, it has been shown that an RPA amplicon can tolerate up to 9 mismatches across all primers and the probe sequences (cf. Boyle et al., 2013, doi:10.1128/mBio.00135-13; Daher et al., 2014, DOI: 10.1016/j.mcp.2014.11.005). This could be an advantage in situations in which the detection of closely-related targets that differ by one or a few bases is required, wherein a prototypic example for this setting would be the use of the reaction for detecting an infectious agent, particularly a viral agent, for which it is known that the infectious agent or pathogen undergoes rapid rounds of mutation. In practice, it would thus be highly favourable if one diagnostic kit will be able to amplify and thus detect the nucleic acid sequence of a virus of interest with high specificity, but at the same time tolerating certain mutations to avoid a false negative result.

In parallel to the biochemical advancements in nucleic acid amplification, also the development of devices including suitable reaction and detection chambers for performing nucleic acid amplification in an optimized manner have steadily developed. A great trend has been the development of microfluidic devices and, in general, a trend of miniaturizing or "nanotizing reaction chambers", i.e., putting them into nanoforms, has been of great interest to decrease the sample volumes and thus the amount of reagents needed and to achieve advantages in biochemical detection, time to result, faster thermal transfer, and to have the potential for automation and integration and, what is more, to have the possibility of multiplexed or multimeric tests. Multimeric in this case means multiplexing in the sense of parallel reactions being conducted in separate (locally distinct) reaction chambers.

As clearly exemplified during the Corona pandemic in 2020, there is still an ongoing global need for providing fast and reliable nucleic acid amplification techniques, which cannot only be performed in specialized diagnostic centres, but which can be performed immediately at the site, where a biological sample from a person to be analyzed is obtained. Further, it has been observed at a global scale that time to result is of paramount importance as unless strict and prophylactic quarantine rules apply, there is a huge risk that an infected, yet symptomless, patient may spread an infectious disease until receiving a (reliable) test result which can currently last anything from 24-36 hours or more.

It is observed that rapid testing for more than one pathogen or target sequence becomes of great importance, for example, to discriminate patients positive and negative for SARS-CoV-2 and the seasonal influenza. For cost reasons, double testing is usually not performed by general practitioners usually being the first physicians diagnosing patients with symptoms of suspected respiratory disease. Also regarding prophylactic testing of potentially contaminated surfaces, or of returnees from travels abroad, it is frequently observed that testing is conducted too late (when symptoms of the disease become apparent), or standardized testing which would allow a targeted decontamination of surfaces etc. is not performed regularly in view of the costs involved for PCR tests. Finally, versatile testing systems and devices are presently missing, wherein the reaction format can be easily adapted in case a given panel of targets to be tested (e.g., seasonal Influenza strains and SARS-CoV-2) is to be adapted at short notice (new emerging virus strain to be added, or to be used alternatively).

It was thus an object of the present invention to provide a new multiplex isothermal nucleic amplification method suitable to and specifically optimized for being conducted under non-laboratory conditions as well as under laboratory conditions in a preferably handheld or at least portable diagnostic device to enable point-of-care diagnostic infections even under a home environment and to provide fast and reliable diagnostic results for diagnosing several potential infections and/or target nucleic acids in preferably one and the same test sample directly applied to the diagnostic device. The device should be configured in such a way that an easily customizable test for at least two target sequences should be provided, wherein the device allows that the biochemical reactions performed can be exchanged easily to be adapted to the diagnostic needs of the customer interested in the relevant results.

### Summary of the Invention

The present invention is as defined in the attached set of claims.

### Brief Description of Drawings

- **Figure 1** (Fig. 1): shows a detection system (10) according to the invention, for which the isothermal method of nucleic acid amplification of at least two target sequences in a biological sample was developed and optimized for. The device is further disclosed in detail in, for example, DE 10 2020 109 744.1.
- **Figure 2** (Fig. 2): shows an embodiment of a set of containers to be used with the detection system of Fig. 1.
- **Figure 3** (Fig. 3): shows a schematic diagram of an alternative detection device.
- **Figure 4** (Fig. 4): shows a schematic block diagram of electric components of the detection device.
- **Figure 5** (Fig. 5): shows a mobile suitcase laboratory (Bodo Koennecke, Berlin, Germany) as further detailed in Example 2 below.
- **Figure 6** (Fig. 6): shows a probit regression analysis for RdRP-, E- and N- RT-RPA assays as described below in the Examples. The limit of detection is 2 RNA molecules/reaction for RdRP RT-RPA assay (grey triangle) and 15 RNA molecules per reaction for E and N RT-RPA assays (black circle).
- **Figure 7** (Fig. 7): Sensitivity, specificity, positive predictive value (PPV) and negative predictive value (NPV) of RdRP, E and N RT-RPA assays in comparison to real-time RT-PCR. N is number of samples as detailed in the below Examples.

### Detailed Description

Even though NAATs, including isothermal amplification strategies, have made enormous progress over the last decades, and further taking into consideration that highly specific primers and probes needed for nucleic acid amplification techniques can meanwhile be provided quickly, the whole nucleic acid amplification infrastructure in the year 2020 is still constricted to testing in specialized laboratories. Further, although multiplexing is theoretically possible and multiplex PCR kits are on the market, the present infrastructure for testing does not allow an easily customizable "test on demand" strategy, as the devices and systems needed for testing presently do not allow a parameter (here: the target to be screened for) to be exchanged on demand - and quickly.

Another problem associated with nowadays testing is that samples to be tested, which may be taken from a human or animal subject, or which may be taken from an abiotic surface, may contain an infectious or harmful agent. To avoid cross-contamination and to decrease the contagious potential, it is thus of utmost importance to decrease the steps between sample extraction and analysis. Finally, the test results should be available as fast as possible.

The present invention now provides an integrated solution based on a multiplex customisability, which can be provided by nanoscale amplification chambers, to allow as many parallel tests as possible, so that bespoke individual test panels can be created. Due to the portable and biochemically fully automatic testing reaction, the tests can be performed in a non-laboratory environment. This will not only relieve the burden of testing laboratories, this can also reduce costs and risks associated with the transport of potentially contagious sample material. Finally, this strategy allows highly reliable and fast testing results, as the biochemistry used for target nucleic acid determination is based on isothermal nucleic acid amplification reactions usually, provided a correct primer and probe design, being known to be superior to antigen-based tests in specificity and sensitivity.

The present inventors now provide a new isothermal nucleic acid amplification strategy perfectly interacting in an isothermal assay, like RPA, and having several advantages over commonly used strategies, as the amplification can be done in a portable amplification device underfield conditions, or in a home environment, outside a fully equipped laboratory. Most importantly, the methods provided herein allow for multiplexing, i.e., the simultaneous amplification of at least two target sequences in any biological sample, which is fast and robust and, relying on a recombinase being tolerant to certain mutations, making the new method a perfect one for routine diagnosis in a clinical setting, or in a home environment, or at an airport etc.

Customizable devices for diagnosis, which can be easily adapted to screen panels of target sequences for specific risk/patient/livestock groups, or an analysis of the contamination of a sample taken from an abiotic area, are urgently needed to provide a reliable and fast differential diagnosis for more than one target nucleic acid sequence in short time to be in a position to draw the respective conclusions from a diagnostic test result in short.

In one aspect of the present invention, there may thus be provided an isothermal method of nucleic acid amplification of at least two target sequences, preferably in a biological sample, or in a sample obtained from a surface, or from any another source (soil, air etc.) potentially comprising a target sequence to be detected, the method comprising the steps of: (a) providing (i) at least one recombinase, optionally: at least one recombinase-assisting factor, and at least one single-strand binding protein, at least one, and preferably at least two target sequence specific primers, at least one DNA polymerase, preferably a strand displacement polymerase, and optionally: providing at least one reverse transcriptase and/or at least one energy regeneration enzyme, together with suitable reaction components, wherein (ii) either at least one co-factor essential for the activity of at least one of the enzymes is not provided; or wherein at least one inhibitory agent blocking the activity of the at least one recombinase is provided; (b) providing at least one biological sample to be analyzed for the presence of the at least one target sequence and optionally providing a preferably sterile extraction kit to obtain the biological sample; (c) optionally: providing at least one probe and optionally providing at least one enzyme activating the probe so that a detectable signal is generated; (d) adding a starting reagent to initiate the amplification reaction; and (e) obtaining the at least one amplified target sequence and/or obtaining at least one detectable signal each signal being indicative of a successful amplification of the respective target sequence amplified, optionally wherein at least one of the above enzymes, more preferably at least two of the enzymes, and most preferably all of the enzymes used are thermostable enzymes having optimum activity at a temperature of above 37°C to about 85°C, preferably of about 39°C to about 60°C, and most preferably from about 40°C to about 55°C.

In one embodiment, at least three, at least four, at least five, at least six, or more than six different target sequences may be amplified, preferably in the same sample used. In this embodiment, the device or system will be equipped with more than one amplification chamber representing a separate reaction environment comprising individual and customizable primers and optionally probes. Each amplification chamber can receive the sample, preferably the lysed or otherwise pre-treated and optionally inactivated (decontaminated) sample individually. Therefore, only one sample extraction is necessary, which significantly decreases the risk of cross-contaminations or infections during the transport of a sample to a laboratory. In the example of a home test, or a test performed at the airport etc. a person potentially infected with an infectious agent can be tested immediately and for a certain relevant panel of possible infectious agents so that further methods (quarantine, medication) can be initiated immediately, as the test provides reliable results - for all multimerically tested samples - in below 20 min.

In one embodiment, the target sequence may be individually selected from the group consisting of a target sequence of an infectious agent, including a target sequence of a virus being selected from Ebola virus, Sudan virus, Marburg virus, Bundibugyo virus, Monkeypox virus, Yellow Fever virus, Zika virus, Japanese Encephalitis virus, Rift Valley Fever virus, Dengue virus, Chikungunya virus, Rabies virus, Influenza virus A (FluA) or B (FluB), including H5N1 and H7N9, Respiratory Syncytial virus (RSV), Adenovirus, including Adenovirus 1, 4. 7, Paraifluenzavirus 3, MERS CoV, SARS-CoV-2, Parvovirus B19, West-Nile virus, Herpes simplex virus (HSV), Varizella Zoster virus (VZV), Norovirus, Bovine Coronavirus, Foot and Mouth Disease virus, Lumpy Skin Disease virus, or including a target sequence of a bacterium, including *Staphylococcus* aureus, including an antimicrobial resistance gene, or including a target sequence of *Mycobacterium ulcerans, Treponema pallidum, Leishmania donovani, Mycobacterium leprae, Fransciella tularensis, Bacillus anthracis, including pacA*/*capC, Clostridum difficile, inlcuding tcdB*/*tcdA, Pan-Ricketsia, Salmonella typhi or paratyphi, Plasmodium falciparum, Mycobacterium avium subs. Paratuberculosis,* a Cytomegalovirus (CMV), or an enterovirus.

In a specific embodiment, the at least at least two target sequences may be provided as part of a systematic panel, wherein the panel is selected from the group consisting of an acute infectious disease panel, a meningoencephalitis panel, a severe respiratory disease panel, an arbovirus panel, an antibiotic resistance gene panel, a country-specific travel returnee panel, a panel specific for a screening during pregnancy, a panel specific for testing individuals before planned or acute hospitalization, a panel for screening for screening for infectious pediatric diseases, including pediatric diseases associated with an erythema, and/or a panel for testing livestock animals.

Based on the needs and skills of a user (medical or public sector), a variety of easily customizable variations is thus possible. For example, a meningoencephalitis panel may comprise amplification chambers individually equipped to detect a target sequence of HSV, VMV, VZV, and, as a fourth chamber, at least one enterovirus.

Pediatricians may need panels for screening for infectious pediatric diseases, including pediatric diseases associated with an erythema, or a panel for screening for relevant diarrheal diseases (Norovirus and Rotavirus) to be in a quick position to treat the pediatric patient correctly and to inform the parents and contact persons accordingly. Another kit of clinical significance may be a severe respiratory infection panel, including FluA, FluB, a SARS virus and/or RSV as targets, the nucleic acid sequences of which are to be individually screened in parallel from one and the same probe or sample. Finally, particularly for returnees from countries where the respective diseases frequently occur, an arbovirus panel may be of interest, inlcuding a Chikungunya virus, a Zika virus and a Dengue virus, and optionally a West-Nile virus target sequence.

Furthermore, the multiplexed test system and the kits and methods as disclosed herein may be particularly suitable to screen individuals as well as potentially contaminated surfaces for antibiotic resistance gene carrying organisms, for example, for methicillin resistant *Staphylococcus aureus* (MRSA).

By doing so, hygiene management in hospitals or homes for elderly people can be significantly improved.

In short, an isothermal DNA/RNA amplification method of the prior art, more specifically a "classical" RPA, usually starts with the binding of a phage-derived T4 UvsX protein as central recombinase, assisted by the T4 UvsY acting as loading factor, to the primers (a forward and a reverse primer) to form a nucleoprotein filament starting the reaction and guaranteeing accessibility of the target nucleic acid to be amplified. The resulting complex so to say "searches" for homologous sequences in a duplex DNA usually forming the target. Once the homology is located, the complex invades the double-stranded (ds) DNA, forming a so-called D-loop structure. One side of this D-loop is double-stranded where the primer hybridises with the template strand, initiating a strand exchange reaction, whereas the other side of the D-loop remains single-stranded. This unwound complementary strand is stabilised by a single-strand binding (SSB) protein, where usually a T4-derived gp32 protein is used. Subsequently, the recombinase disassembles from the nucleoprotein filament and becomes immediately available to initiate another strand displacement reaction with a new primer etc. pp. Primer incorporation allows the DNA polymerase, where usually a *Bsu* (*Bacillus subtilis*-derived) or *Sau(Staphylococcus* aureus-derived) polymerase, usually the large fragment of a polymerase I, is used, to initiate the synthesis from the free 3'-OH at the end of the primer. As the polymerisation continues, the two parental strands will thus continue to separate. Incorporation of both forward and reverse primers then enables strand synthesis to occur in both directions simultaneously, and ultimately results in the exponential accumulation of amplified duplex DNA, consisting of the sequence between the forward and reverse primers.

RPA was initially demonstrated to be a nucleic acid amplification method for DNA. In turn, and as of particular importance also for the methods as disclosed herein, RPA was later also shown to be a suitable reaction for RNA as template by addition of an additional enzyme, a reverse transcriptase (e.g., from Murine Leukemia virus (MuLV) reverse transcriptase) in the same reaction compartment. Regardless of nucleic acid template type, the recommended RPA amplicon length should be below 500 nucleotides for efficient amplification. Most presently published RPA papers have applied amplicon lengths between 100 and 250 nucleotides, which usually incur fast and efficient amplification. However, shorter amplicons (79 nucleotides; 94 nucleotides and longer amplicon up to 1,500 nucleotides have also been reported (see Lit et al., Analyst, 2019, supra*).* For the purpose of the present invention, a short amplicon length within the limits of a usual RPA reaction will usually be preferable, as it increases the efficiency of the amplification. Still, the design of primers and optionally a probe will be of particular importance when the methods as disclosed herein are performed to provide reliable diagnostic results. Unlike for PCR, the length of RPA primers is usually relatively long (a recommended minimum of 30 nucleotides, but it will range between about 32 and 35 nucleotides). Shorter PCR primers (typically between 18 and 25 nucleotides) can also be used in the RPA reaction but may decrease the reaction speed and sensitivity, which is to be avoided in diagnostic settings. In preferred embodiments according to the methods of the present invention, particularly if used for diagnostic purposes, the general amplicon length, which can be influenced by the corresponding primer design, will usually range from about 50 to about 400 base pairs (bps), preferably 100 to about 200 bp, and most preferably about 120 to about 150 bps. An amplicon length below 200 bps is considered to be favorable for diagnostic purposes as the length of the amplicon has an influence on the efficiency of the amplicon amplification and, therefore, is directly linked to the analytical sensitivity of the reaction. Inefficient longer amplicons also result in slightly longer reaction times, which generally should be avoided. In preferred embodiments, at least one, at least two, at least three, at least four or even all enzymes of the method of the present invention are thermostable enzymes.

Historically, RPA assays were established with phage derived enzymes with a focus on the functionality, i.e., the specific catalytic activity, of these enzymes. "Standard" RPA enzymes, even nowadays, are usually derived from mesophilic phages like T4 or T6 (cf. Li et al., 2019, *supra),* i.e. phages specific for the gut bacterium *Escherichia coli* and thus mesophilic in the sense that the bacterium and thus the phage lifecycle is adapted to standard temperatures (around body temperature). Still, these enzymes have certain disadvantages as these enzymes are not specifically thermostable, even though these enzymes due to their functionality are commonly used in methods requiring temperatures above 40°C. "Thermostability" or "thermostable" as used herein is meant to describe the quality of folded protein or enzyme to resist irreversible change in its chemical or physical structure, for an enzyme its three-dimensional folded state active as biocatalyst, by resisting decomposition or polymerization, at a high relative temperature above standard temperature, wherein the relevant reference temperature representing a standard temperature is 37°C. A "thermostable enzyme" as used herein can thus be characterized by showing denaturation only at temperatures above around 70°C, 71°C, 72°C, 73°C, 74°C and preferably above 75°C and/or an optimum activity temperature at 37°C to about 85°C, preferably of about 38°C to about 80°C, and most preferably from about 40°C to about 65°C. With this optimum activity temperature, the at least one enzyme will be perfectly suited for a method performed in a portable diagnostic device as disclosed herein to provide fast and reliable results. Finally, the thermostable enzyme may have an optimum pH at or around a physiological pH, but this is not strict prerequisite. In certain embodiments, the optimum pH will be in a range from about pH 6.0 to about pH 9.0, more preferably in a range from about pH 6.5 to about pH 8.5, and most preferably in a range from about 7.0 to 8.0. It should be guaranteed that the enzymes chosen to interact in the same reaction environment will be provided in a suitable buffer system so that all enzymes can be active and will exert their catalytic activity.

To provide cheap and readily customizable testing devices or systems, a multiplexing or multimeric compatible system as used herein can be equipped with any kind of chemistry supporting an isothermal nucleic acid amplification method. If massively parallel amplification in various amplification chambers is needed, it may be suitable to use established and easily available enzymes.

In certain embodiments, the use of thermostable enzymes may allow an easier temperature control of an isothermal nucleic acid amplification reaction in comparison to a standard RPA reaction though. Further, the reaction can be performed in a better controllable manner and the enzyme stability after storage and reconstitution - an important prerequisite for enzymatic activity and thus a reliable and fast test result - can be guaranteed. Finally, the reaction additives can be drastically reduced further improving the reaction. In contrast to an RPA, for example, the addition of a crowding agent necessarily used in standard RPA assays can be avoided or reduced to a minimum. In a preferred embodiment, no crowding agent is separately added to an isothermal nucleic acid amplification reaction according to the present invention going beyond inevitable impurities of a crowding agent (e.g., PEG or glycerol) as used during the lyophilization process. For all methods as disclosed herein, the skilled person will recognize that for any thermostable enzyme as disclosed herein, a thermostable functional equivalent from another organism, or a recombinantly modified mutant thereof, may be used as well without deviating from the gist of the present invention.

A "biological sample" is to be understood broadly and relates to any material isolated from a living organism, which contains genetic material from this organism and/or (additionally) from a pathogen (a pathogenic prokaryote or eukaryote) or virus (with an RNA or a DNA genome, single, or double-stranded, or a retrovirus) having infected or infiltrated the organism, or living in symbiosis with this organism etc. A biological sample includes, for example, swab and saliva samples, including nasopharyngeal and oropharyngeal swab or saliva, but also sputum, blood, urine and stool and the like. Preferably, a biological sample to be tested in a non-laboratory environment will be a swab, saliva, urine, or stool etc. which can be retrieved without the assistance of trained medical personnel.

Notably, the present invention is also perfectly suitable to detect at least two target nucleic acid sequences from a sample obtained from an abiotic surface, but potentially being contaminated with, for example, a MRSA.

Suitable reaction components for enzymatic reactions and the concentration and compounding thereof, in case the enzyme and the reaction these suitable reaction components are intended for is known, can easily be determined by the skilled person. Certain aspects of particular relevance for the methods of the present invention will be disclosed in detail in the following. Particularly, the necessary primers and optional the probes of interest for each of the target sequences of interest to be analyzed can be provided by the skilled person based on the disclosure as provided herein and based on the skilled person's common general knowledge. In view of the fact that genomic sequences of the relevant infectious agents and/or the sequence of various antibiotic resistance genes (cf. Sabino et al., Nature Comm., 10, 5252 (2019)) are available online, the skilled person can use the relevant information and add suitable primers and/or probes to a system as disclosed herein for use in a method as disclosed herein.

A "starting reagent" as used herein according to the various methods, can be particularly suitable, as this reagent allows an exact and controllable start of the reaction. A starting reagent can be an essential co-factor of at least one enzyme, without which the enzymatic reaction cannot start. For example, the starting reagent may be magnesium, or a salt thereof or a magnesium containing solution, for example, magnesium acetate. The concentration can vary and can be between around 5 nM and 20 mM. The starting reagent may be provided in an activatable form, in a separate compartment, or in a heat activatable manner so that the start of the reaction can be tightly controlled. In case the method of the present invention is to be performed in a non-laboratory environment, the starting reagent will be provided in a manner that the reaction can be started with a simple action of the user (click on a button, or automatic start after application of a test sample).

Further, a nucleic acid amplification reaction will require dNTPs (nucleotide triphosphates), for example, dATP, dGTP, dCTP, and dTTP. In leading and lagging strand amplifications, ATP, GTP, CTP, and UTP may also be included for synthesis of RNA primers. In addition, ddNTPs (ddATP, ddTTP, ddGTP and ddGTP) may be used to generate fragment ladders. The dNTP may be used at a concentration of between 1 µM to 200 µM of each NTP species. A mixture of dNTP and ddNTP may be used with ddNTP concentrations at 1/100 to 1/1000 of that of the dNTP (1µM to 200 µM). The use of UTP may be particularly suitable in case a carry-over prevention kit (e.g., Thermo Fisher using a so-called AmpErase) is used. In these embodiments, a suitable, usually recombinant, uracil N-glycosylase (UNG) can be used to prevent that an amplification product is again amplified in a subsequent reaction. To this end, the reaction can perform faster and false positive results can be avoided.

Further, a reducing agent may be used, wherein the reducing agents to be used includes dithiothreitol (DTT). The DTT concentration may be between 1 mM and 10 mM.

Obviously, the methods as disclosed herein are not only suitable to amplify DNA, but also to amplify RNA and thus to detect RNA virus material in a biological sample of interest.

In certain embodiments, a probe may be used in the methods as disclosed herein, particularly in those methods performed on the diagnostic device as disclosed and claimed herein, which is specifically configured to comprise different light sources and light sensors to detect at least one probe-mediated signal, and preferably at least two different probes: one associated with the target nucleic acid to be amplified, and one associated with a positive control. The use of reliable positive controls, particularly in the field of diagnosis, more particularly the diagnosis of infections agents like SARS-CoV-2, is very helpful and not included in presently available test kits, as the positive control allows a conclusion whether the reaction as such worked. This is of particular importance and a huge advantage in case the test is not performed by trained experts in a laboratory, but in a home environment, or in test stations.

The term "probe" as used herein broadly refers to a molecule or atom used in molecular biology, and particularly in the methods as disclosed herein, to study the properties or, for diagnostic setting, the quantity of another molecule or structure of interest. A variety of different probes is available to the skilled person, which probes can be easily combined with the methods as disclosed herein (cf. Molecular Probe Techniques, Tree Number(s) E05.601, Unique ID D015336, RDF Unique Identifier, http://id.nlm.nih.gov/mesh/D015336). For using the methods as disclosed herein on the specific device as disclosed herein, probes, optionally activatable probes, providing a detectable and/or quantifiable signal may be preferred, e.g. Forster/fluorescence resonance energy transfer (FRET) probes etc. In view of the special configuration of the device as disclosed herein, this may allow a rapid diagnostic result. As different light sources, sensors and detectors are provided, this offers the advantage to include a positive control associated with one probe, and a different probe associated with the actual target sequence, which may drastically increase the reliability of a diagnostic result.

In the context of the present invention, any luminescent detection system may be used to detect an amplification result of interest fast and reliably. Luminescence is the umbrella term for any kind of spontaneous emission of light by a substance not resulting from heat. Luminescence comprises various types of luminescence, wherein the most relevant for the purpose of the present invention are chemiluminescence, including bioluminescence and electroluminescence as well as photoluminescence, including fluorescence and phosphorescence, as a device as disclosed herein can be equipped appropriately to detect several kinds of luminescent probes or analytes easily.

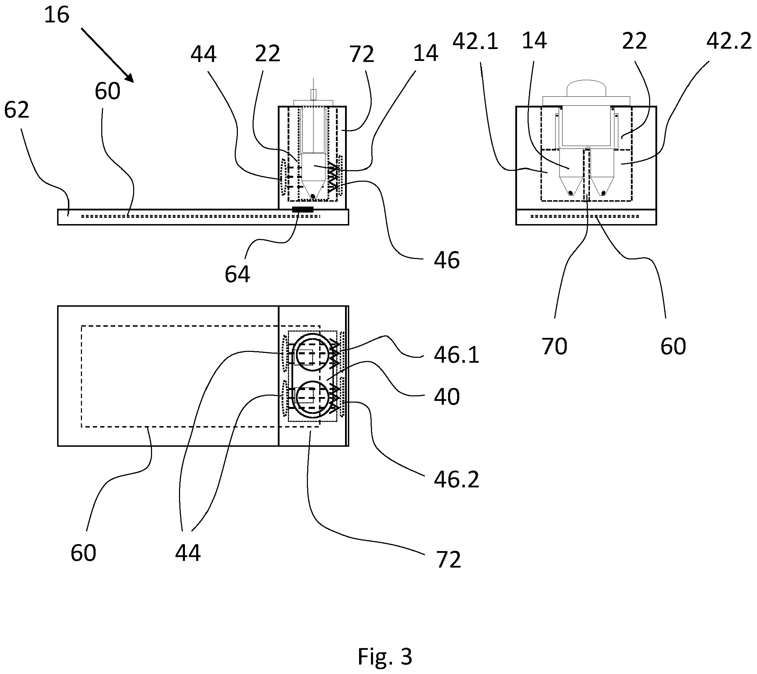

Probes that can be incorporated during the RPA reaction include, for example, a commercially available TwistAmp^{™} exo probe (typically having a length of about between 46 and 52 nucleotides) and the TwistAmp^{®} fpg probe(typically between 32 and 35 nucleotides), These probes are used for fluorogenic real-time detection. These two probes are usually labelled with a fluorophore, a quencher (e.g., a BlackHole Quencher) that is in close proximity to the fluorophore, to temporarily deter the fluorescent signal, and a blocker (e.g.C3-spacer, a phosphate, a biotin-TEG or an amine) at the 3'-end serving to prevent polymerase extension from the 3'-end (see again Li et al., Analyst, 2019, supra, particularly Fig. 3A and B). The real-time detection is based on cleavage of fluorogenic probes at an abasic site (also known as an apurinic/apyrimidinic site that is a location in DNA (less often in RNA), which has neither a purine nor a pyrimidine base) between the fluorophor and the quencher. The abasic site can either be tetrahydrofuran (THF) or a dSpacer (a derivative of the THF) or a dR group (the deoxyribose of the abasic site via a C-O-C linker). The E. *coli* exonuclease III, for example, cleaves the TwistAmp^{™}exo probe at a THF or a dSpacer site, while the *E*. *coli* glycosylase/lyase *E*. *coli* cleaves the TwistAmp^{™} fpg probe at the dR position. After the enzymatic cleavage, the TwistAmp^{®} exo probe can serve as a forward primer. However, the TwistAmp^{™}fpg probe cannot serve as a primer due to different catalytic mode (beta-elimination) of the *E*. *coli* glycosylase/lyase fpg protein, which does not generate an extendable 3'-OH group but a 3'-phosphate group. A variety of different probes or labels for marking a nucleic acid base or a nucleotide of interest with a detectable label, including probes not necessitating the addition of at least one activating enzyme, are imaginable as long as there is at least one suitable light source and a cognate detection device specifically detecting the presence of the probe and/or label by producing a detectable signal.

In another embodiment, a further luminescent luminophore dye may be used (cf. Roy et al., Biosens Bioelectron. 2016 Dec 15; doi: 10.1016/j.bios.2016.06.065.).

All primers or probes as used in the methods as disclosed herein can be naturally occurring DNA and/or RNA sequences, or synthetic primers or probes including naturally occurring bases and/or backbones, or synthetic elements including labels covalently and/or non-covalently attached to the primer or probe, or including at least one phosphothioate backbone for increasing the stability of the primer or probe, or to improve the amplification of DNA sequences by DNA polymerases with proofreading activity (Skerra, Nucleic Acids Res 20, 3551-3554, doi:10.1093/nar/20.14.3551 (1992)), and any combination thereof.

The device according to the present invention in its overall configuration can be easily adapted to be suitable to detect various fluorophores, chromophores, luminophores or other probes and/or analytes or dyes to be visualized by a light source. In a specific embodiment, the visualization will be one caused by a pH drop occurring during the amplification reaction, which - provided that suitable pH-sensitive dyes are added to the reaction mixture, for example, phenol red or crescol red - allow a colorimetric distinction between positive and negative results with the naked eye. In this case, the detection chamber (42) may be configured to comprise a transparent window so that the result of an amplification reaction as evidenced by a simple colour scheme (one colour = positive; another colour = negative) can be easily retrieved with the naked eye. Possible techniques to be easily adapted for the purpose of the present invention are disclosed in Huang, et al., Microb Biotechnol 13, 950-961, doi:10.1111/1751-7915.13586 (2020), or in Lu, et al., Int J Mol Sci 21, doi:10.3390/ijms21082826 (2020).

A "crowding agent" as used herein is meant to specify any agent assisting in providing an optimum reaction environment for an enzymatic reaction. The mode of action of a crowding agent is the creation of smaller reaction compartments so that the probability of a reaction between a substrate and the cognate enzyme is increased. Crowding agents usually actively added to a RPA reaction include, for example, polyethylene glycol (PEG), dextran and ficoll. The crowding agent may usually be at a concentration of between 1% to 12% by volume or 1% to 12% by weight of the reaction. While all PEGs are useful, preferred PEGs were disclosed to include PEG1450, PEG3000, PEG8000, PEG10000, PEG compound molecular weight 15000-to 20,000 (also known as Carbowax 20M), and a combination thereof. Still, the addition of an additional agent like a crowding agent adds more complexity to a reaction. In the methods as disclosed herein, the thermostable nature of the enzymes used for the amplification method and further the fact that the specific device for performing the amplification reaction allow an optimum reaction environment, wherein the reaction partners are brought together with an analyte, the biological probe to be analyzed, in a specialized diagnostic device and/or the fact that the enzymes can be used at elevated temperatures without losing activity, the reactivity of components generally increasing with an increase in temperature. Therefore, the methods of the present invention can proceed without the active addition of a crowding agent.

Alternatively or additionally, nanostructured elements may be used in the reaction mixtures of the present invention to further optimize the amplification result, for example, ZnO nanostructures (Ma et al., Virus Res., 2017, 232: 34-40. doi: 10.1016/j.virusres.2017.01.021.).

The buffer solution in an isothermal method of the present invention may be a Tris-HCI buffer, a Tris-Acetate buffer, or a combination thereof, but other buffer systems are suitable as well. The buffers may be present at a concentration of between around 10 nm to 100 mM, which will depend on the buffer system used and the pH of the reaction to be achieved. The latter is influenced by the optimum activity of an enzyme of interest, as it is known to the skilled person.

All thermostable enzymes as tested for the purpose of the present invention were chosen in a way to have a rather homogenous optimum pH activity range between around pH 6.0 to about 9.0, preferably in the range of pH 7.0 and 8,5. The buffered pH may thus be between 6.0 to 9.0. The buffer may further contain Mg ions (e. g., in the form of Mg Acetate) at a concentration between 5 nm to 100 mM with a concentration of between 5 to 15 mM being preferred. One preferred Mg concentration is 10 mM (Mg concentration or Mg Acetate concentration).

Usually, an RPA reaction may be incubated between 5 minutes and 16 hours, such as between 15 minutes and 3 hours or between 30 minutes and 2 hours. The incubation may be performed until a desired degree of amplification is achieved (cf. EP 2 336 361 A2). The huge advantage of the methods as disclosed herein is the fact that these methods are extremely fast in amplification in view of the special configuration of the thermostable biochemical components used in a specific portable system or device combined with certain detection techniques as enabled by at least one luminescence, preferably at least one fluorescence signal as detectable by the portable system. Therefore, the method after a few minutes, e.g., after 2 min, after 3 min, after 4 min, after 5 min, after 6 min, after 7 min, after 8 min, after 9 min, or at latest after around 10 min provides a reliable result for a positive control and, in turn, for a biological sample to be analyzed as positive or negative.

In certain embodiments of the methods of the present invention, at least one recombinase-assisting enzyme or factor (a chemical) may additionally be provided, preferably before step (d). Such an enzyme or chemical fulfils the function of a co-enzyme of the major working horse, the recombinase and/or the chemical factor activates, stabilizes or otherwise enhances the activity of the recombinase. For RPA reactions, UvsY is such a recombinase-assisting enzyme or loading factor helping to start nucleoprotein filament building, or to stabilize the nucleoprotein complex with recombinase any kind (Li et al, *supra,* or EP 1 759 012 A2). Another recombinase-assisting enzyme of interest may be one that can promote efficient disassembly of recombinase/dsDNA complexes after DNA synthesis initiation. These auxiliary enzymes include those that are capable of stimulating 3' to 5' disassembly and those capable of supporting 5' to 3' disassembly. Further recombinase-assisting enzymes may include several polymerases that can displace RecA or an equivalent, preferably thermostable, recombinase in the 3' to 5' direction and can stimulate 3' to 5' disassembly of recombinase-dsDNA complexes. These DNA polymerases include preferably thermostable equivalents of *E*. *coli* PolV and homologous polymerase of other species. Other recombinase-assisting enzymes include a class of enzymes called helicases which can be used to promote the disassembly of a recombinase from dsDNA. These promote disassembly in both the 5' to 3' and 3' to 5' directions. Helicases are essential components of the recombination process *in vivo* and function to move the branch points of recombination intermediates from one place to another, to separate strands, and to disassemble and recycle components bound to DNA. Still further recombinase-assisting enzymes include *E*. *coli* RecG homologoues and preferably thermostable enzymes. RecG can stimulate disassembly of branch structures. In its natural environment, this enzyme functions to reverse replication forks at sites of DNA damage by unwinding both leading and lagging strands driving the replication fork back to generate a 4-way junction.

For SIBA-type reactions, a sucrose phosphorylase may be provided as additional recombinase-assisting enzyme (see Hoser et al., *supra).*

The central thermostable enzyme of the methods as disclosed herein is a recombinase, preferably a thermostable recombinase (cf. SEQ ID NO: 1). A recombinase is an enzyme that can coat single-stranded DNA (ssDNA) to form filaments, which can then scan double-stranded DNA (dsDNA) for regions of sequence homology. When homologous sequences are located, the nucleoprotein filament (comprising the recombinase agent) strand invades the dsDNA creating a short hybrid and a displaced strand bubble known as a D-loop. Recombinases known for long include the E. *coli* RecA protein or any homologous protein or protein complex from any phyla. These RecA homologues are generally named Rad51 after the first member of this group to be identified. Other recombinase agents may be utilized in place of RecA, for example as RecT or RecO. Recombinase agents generally require the presence of ATP, or other nucleoside triphosphates and their analogues to be active.

Also for the methods as disclosed herein, it is preferred that at least one thermostable recombinase agents is used in a reaction environment under suitable reaction conditions in/under which regeneration of targeting sites can occur shortly following a round of D-loop stimulated synthesis. This will avoid a stalling of amplification or inefficient linear amplification of ssDNA caused by oscillating single-sided synthesis from one end to the other.

In one embodiment of the methods as disclosed herein, at least one and preferably at least two, and most preferably all of the enzymes provided may be independently selected from the group consisting of SEQ ID NO: 1 to 8, or a catalytically active fragment thereof, or a sequence having at least 90%, 91%, 92%, 93%, 94%, 95%, 96%, 97%, 98%, or at least 99% to the respective reference sequence, or a nucleic acid sequence encoding the same.

In another aspect, there may be provided a thermostable enzyme, or preferably a combination of at least two thermostable enzymes for use in a method of isothermal nucleic acid amplification, wherein the at least one enzyme is independently selected from the group consisting of SEQ ID NO: 1 to 8, or a catalytically active fragment thereof, or a sequence having at least 90%, 91%, 92%, 93%, 94%, 95%, 96%, 97%, 98%, or at least 99% to the respective reference sequence, or a nucleic acid sequence encoding the same. For multimeric or multiplexing methods, also conventional enzymes having a comparable functionality as the thermostable enzymes for isothermal nucleic acid amplification as disclosed herein may be used.

In yet another embodiment, there is provided a functional equivalent of an enzyme according to any one of SEQ ID NOs: 1 to 8. As it is known to the skilled person, the sequence of bacterial, viral and archaea sequences may only be poorly conserved during evolution. A "functional equivalent" in this context as used above thus is intended to refer to an enzyme, which is thermostable and has a comparable function, meaning an enzymatic function or catalytic activity, as the respective enzyme of any one of SEQ ID NOs: 1 to 8 a functional equivalent directly compared to. Of course, these functional equivalents may also be used according to the methods of the present invention.

As detailed below, the sequences of SEQ ID NOs: 1 to 8 were identified after *in silico* studies searching for candidates for temperature- and non-laboratory environment conductible isothermal methods for nucleic acid amplification. The aim was to obtain a set of enzymes working fast and reliable, but with a better robustness so that the reaction can be easily be conducted also by untrained personnel outside a lab. Surprisingly, the candidates as identified showed a very promising enzymatic activity, if used alone, or in combination with standard enzymes used in RPA/SIBA reactions, but the enzymes and the reaction *in toto* was much better suited for the non-lab use. Based on the fact that the enzymes are more robust and thermostable, certain agents and steps of classical RPA/SIBA reactions can now be skipped. Reducing the complexity of the reaction and enhancing the temperature (without a loss in sensitivity etc.) is of great importance for the diagnostic use, and even more for the non-laboratory diagnostic use. Further, the production costs can be reduced if decreasing the complexity of the biochemistry driving the reaction.

In a further embodiment of the methods as disclosed herein, at least one energy regeneration enzyme system is provided. Generally, as described, the presence of ATP as energy source for the amplification method as disclosed herein is relevant. An ATP regeneration system was earlier described as crucial for RPA reactions to permit persistent recombination reactions as recombinases have an extremely high rate of ATP hydrolysis when bound to nucleic acids (cf. EP1 789 012 A2). In particular, the UvsX protein was described to have a hydrolysis rate 10-20 times higher than recA and can consume 200 molecules of ATP per minute per monomer. A number of systems are available and are meanwhile routinely used for recycling energy in isothermal RPA reactions and similar reactions, as poor regeneration of ATP may reduce the reaction rate and likely stop the reaction before detectable levels of product have One of those systems is the creatine kinase/phosphocreatine system, or chicken myokinase, which converts a molecule of AMP and one of ATP to two molecules of ADP. ADP is then converted to ATP using the creatine kinase/phosphocreatine system (Li et al., *supra).* For these enzymes, a phosphocreatine di(tris) salt or buffer may be suitable (1 mM to 100 mM).

The present inventors found that the provision of an energy-regeneration enzyme or system may be perfectly compatible with the methods as disclosed herein using thermostable enzymes. Still, it was also found that providing a simple surplus of ATP will perfectly suffice to perform a reliable amplification reaction in a single vial in short time providing a reliable result. Therefore, particularly if a kit and/or a diagnostic handheld system of the present invention is used to perform the methods as disclosed herein it may be preferable to provide a surplus of ATP in an amount above 1 mM to 100 mM. For preparative purposes (e.g., DNA synthesis), a higher concentration of ATP will be needed than for a diagnostic purpose, where a defined amount of a rather short fragment between two primers will be amplified. It was found that providing a high concentration of ATP will suffice to drive the amplification reaction successfully, which can be preferable for several reasons: (i) the reaction will be cheaper, as no additional enzymes will be needed, and (ii) the system will be less complex and thus more stable and (iii) robust in its use, which will be a particular advantage if used in a home environment and when performed nearly automatically, and not by a trained expert in a laboratory field.

In certain embodiments of the methods as disclosed herein, at least one enzyme comprises at least one tag and/or label.

Protein tags, i.e., artificial sequences added to the coding sequence of a protein or enzyme of interest, are well familiar to the skilled person in the relevant technical field of biotechnology and molecular biology. These tags are added to a protein to facilitate the purification after the recombinant production thereof (so-called affinity tag), the visualization, the solubility of a protein etc.

According to the various embodiments of the present invention, every enzyme used according to the methods as claimed and disclosed herein may comprise at least one tag, either at the N-terminus, or at the C-terminus, or also within the protein sequence (without disturbing the function of the protein, preferably between to individual domains, of present), or any combination thereof (e.g., N- terminal and C-terminal tag, which will usually be different, e.g. two different affinity tags, or the combination of a solubility tag and a visualization tag etc.). The term "tag" or "protein tag" thus refers to a variety of different polypeptide sequences or the corresponding nucleic acid sequences encoding the same, which can be incorporated into a protein of interest at various positions, preferably at the N- and/or C-terminus, or between distinct functional domains, and fulfil several different functions. Protein tags can, for example, be differentiated into affinity tags such as chitin binding protein (CBP), maltose binding protein (MBP), Strep-tag and glutathione-S-transferase (GST) or His-Tag. Affinity tags can be used for specifically purifying the protein of interest. Another example of protein tags are solubilization tags, which allow to obtain the protein of interest in its soluble form especially in bacterial cell culture. Epitope tags can be used for having a binding site for analytically antibodies and luminescence, preferably fluorescence tags offer the possibility to detect the protein of interest in the cell culture. A tag or label can also serve the purpose of labelling a protein or enzyme, or a fragment thereof, of interest to allow an easy identification. This may be of particular interest in diagnostic purposes.

In certain embodiments, at least one thermostable enzyme as disclosed herein will preferably not comprise an affinity tag at all, as the thermostable enzyme can be produced and purified without the use of affinity tags. Even though having significant advantages like, for example, enhanced protein purification via the affinity tag whilst having no or low impact of the tag on protein function, particularly affinity tags may have the disadvantage that the tag might have an impact on protein activity so it must be tested individually at which position the tag has only minor impact on protein activity. Further, tags demand high material costs for affinity chromatography. Additionally, some tags may lead to protein inactivation. Even if cleavable tags may be used, this will cause additional efforts, as a suitable protease will have to be added, which will then have to be removed again in a separate process step.

A "label" can be a chemical modification or isotope substitution that makes possible the identification of a molecule, moiety or atom throughout its metabolic transformations or transport, or particularly during the course of a diagnostic or preparative assay of nucleic acid amplification.

Standard suitable reaction conditions and concentrations of agents for the isothermal methods as disclosed herein (e.g., enzymes, primers, probe, dNTPs, ATP, buffers, pH, etc.) are known to the skilled person and cancan be taken from, for example, Li et al., Analyst, 2019, *supra,* whereas special conditions to be taken into consideration are disclosed herein for special embodiments.

In one embodiment of the method of the present invention, wherein at least one forward and at least one reverse primer are provided as target sequence specific primers, wherein at least one of these primers, preferably the reverse primer, is provided in a higher amount or concentration in relation to the other primer. Usually, primers, or a set of primers, or several set of primers, of a multiplex analysis is of interest, can be provided according to the methods as disclosed herein, wherein the primers are usually provided in an equimolar ration. Still, it was found out that for certain purposes, e.g., for the diagnostic of a RNA virus, it may be suitable to provide a higher ratio of one primer (depending on the fact whether it is a (+) or (-) ssRNA virus) to obtain more cDNA for the following amplification step, which will increase the performance of the reaction.

In certain embodiments, the methods of the present invention may be used for a target sequence, which is a single stranded or double stranded (c)DNA and/or RNA nucleic acid sequence. In turn, the method can be used for all kinds of amplification reactions on all kind of nucleic acid material.

As it is known in the field of molecular detection techniques, comprising the use of PCR and of isothermal methods, there are various ways of visualizing the result of an amplification reaction. These techniques are known to the skilled person and can be used for the methods as disclosed herein.

In one embodiment, for example, the at least one primer, including single-stranded and partially double-stranded primers, is itself labelled with a detectable label. It should be noted that a fluorescence quencher is also considered a detectable label. For example, the fluorescence quencher may be contacted to a fluorescent dye and the amount of quenching is detected. Not to interfere with the reaction, the detectable label should be such that it does not interfere with the amplification reaction. Where the primer is partially double stranded with an invading strand and a non-invading strand, the detectable label should be attached in such a way so it would not interfere with the amplification reaction of the invading strand. The non-invading strand of a partially double stranded primer is not elongated so there are no limitations on the labelling of the non-invading strand with the sole exception being that the label on the non-invading strand should not interfere with the elongation reaction of the invading strand. Labelled primers offer the advantage of a more rapid detection of amplified product. In addition, the detection of unincorporated label, that is, labelled oligonucleotides that have not been extended, will allow the monitoring of the status of the reaction.

In another embodiment, a double stranded primer may be labelled such that the separation of the two strands of the primer may be detected. As discussed above, after multiple rounds of amplification, the invading strand and the non-invading strands of a partially double stranded primer is separated. After this separation, the non-invading strand does not participate in the amplification reaction, which in turn may be used to detect and monitor the result of the amplification reaction on-line.

In certain embodiments of the methods as disclosed herein, at least one probe and/or label is provided, wherein the at least one probe and/or the at least one label comprise a directly or indirectly detectable moiety.

Further, the detectable label may be a fluorescent label or an enzyme and the label quencher (also referred to as the label inhibitor) may be a fluorescence quencher or an enzyme inhibitor. In these cases, the label is detected by fluorescence or enzyme inhibition. The delectability of the label would be the fluorescence if a fluorescent label is used or enzyme activity if an enzyme is used.

In a preferred embodiment, a separate probe is provided, which probe may be target sequence specific. Further, the probe may serve the purpose to provide a detectable or visible signal to monitor the result of the amplification reaction in a convenient manner. The probe may carry an activatable label or signal-giving component, which can be activated, for example, by the addition of an enzyme, for example an exonuclease III (Exo III, exo herein) as used in Twist-RPA reactions. The label may also be associated with at least one primer, in certain embodiments. Even though the detectable moiety may be selected from the group consisting of a fluorochrome, an enzyme, a fluorescence quencher, an enzyme inhibitor, a radioactive label, a chromogenic agent, and a combination thereof, for a non-laboratory use of the methods of the present invention in a system as disclosed herein, the use of radioactive material and/or generally the use of hazardous substances (chemical and/or biochemical agents) should be avoided.

In a further aspect of the present invention, there is provided a system for detecting at least one target sequence in a biological sample, or in a sample obtained from a surface, preferably a portable system, for example, a portable diagnostic system or device, said system comprising: at least one lysis chamber (12), at least two amplification chambers (14; 14.1 and 14.2, for example) and a luminescence, preferably a fluorescence detection device (16), wherein the at least one lysis chamber (12) contains a lysing fluid, the amplification chamber (14) contains a mixture that comprises the set of enzymes and the suitable reaction components as defined in claims 1 to 10, and the luminescence, preferably the fluorescence detection device (16) comprises a detection chamber (42) configured to receive the amplification chamber (14) or the contents of the amplification chamber, a light source (44), an optical sensor (46), an energy supply means (48), a wireless data interface (52) and a controller (50), wherein the system is configured to perform a method according to any one of claims 1 to 10 and thus allows for diagnostic multiplexing analysis in parallel.

In one embodiment, the system may the system may be configured so that it can be combined to form a single, fluid tight assembly (34), wherein the at least one first lysis chamber (12) comprises a first set of chemicals and/or agents, said first chamber being closed prior to use, preferably wherein the first set of chemicals and/or agents allows the accessibility of the at least one target nucleic acid sequence to be amplified and/or the inactivation of potentially contagious material in a sample, and wherein a second amplification chamber (14.1 or 14.2) of the at least two amplification chambers (14) each comprises a second set of chemicals and/or agents that are at least in part distinct from the chemicals and/or agents of the first set and being at least distinct from the chemicals and/or agents of the at least one further second amplification chamber set in that parts decisive for target gene specificity, and wherein the at least one first lysis chamber comprises a lid (26), that can be opened when the at least one first lysis chamber (12) and one of the at least two second chambers (14.1 or 14.2) are combined to form a single, fluid tight assembly (34), in order to allow the contents of the at least one first lysis chamber (12) to individually enter each of the at least two second chambers (14).

It is thus a huge advantage of the present system that a potentially dangerous sample to be tested for the presence of at least two nucleic acid target sequences of interest only has to be extracted and provided to the system or device as disclosed herein once. Furthermore, the sample can be obtained or extracted directly in situ at the place where the amplification reaction and the subsequence diagnosis is performed. By doing so, this of course drastically reduces the risk of contaminations or a spread of a potentially infectious or dangerous sample, as the sample does not have to be transported to a laboratory. The multiplexing approach thus allows that a sample of interest is directly distributed over the amplifications chambers, wherein each amplification chamber comprises the necessary agents to detect a target sequence of interest.

This specific arrangement and the configuration of the system allows the huge advantage to include a control reaction, e.g., a positive control, for which usually no separate lysis may be necessary, if a standardized control is provided, in case the system is used as diagnostic device. For this use, the different compartments or chambers of the system are particularly suited to perform a method of the present invention together with the kit components of the present invention, said kit components preferably being provided together with the system, so that only the biological sample to be analyzed has to be added. In another embodiment, the set of chambers can be even increased to provide a tightly controllable diagnostic device providing parallel multiplex analysis, and at the same time providing high hygienic standards.

In one embodiment, the system may additionally comprises a luminescence, preferably a fluorescence detection device (16), the luminescence, preferably the fluorescence detection device (16) comprising: a detection chamber (42) configure to receive an amplification chamber or the contents of the amplification chamber, a light source (44) configured to excite luminescence and in particular fluorescence, an optical sensor (46) configured to capture luminescence and in particular fluorescence, energy supply means (48), a wireless data interface (52) and a controller (50).

This embodiment may be perfectly combined with the methods of the present invention, wherein a fluorescent, or an activatable fluorescent, probe or primer is used so that the amplification product will produce a detectable signal immediately showing a quantitative and qualitative result of the reaction. The system will preferably comprise a luminescence, preferably a fluorescence detection device allowing the identification of different luminescent signals so that at least two signals, e.g., a potential signal of a probe to be analyzed, and a signal related to a standardized positive control, can be detected.

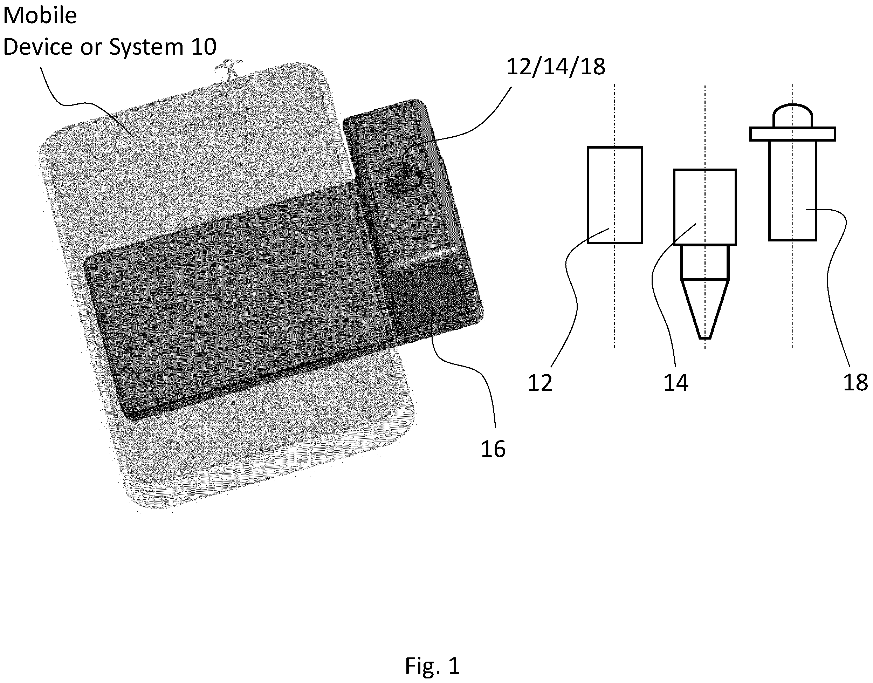

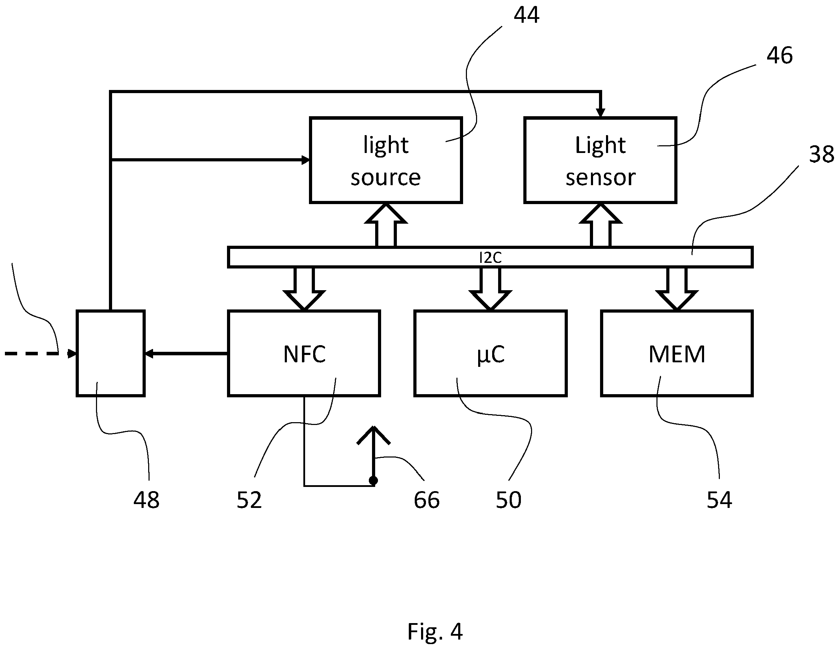

Importantly, the methods for isothermal nucleic acid amplification as disclosed herein have been specifically optimized to be compatible with a system as designed by the present applicant, the system or device being suitable for rapidly detecting a target DNA or RNA in a biological sample, also under non-laboratory conditions. This system of the present invention as particularly suitable for performing the methods as disclosed herein can be described as follows and with reference to the attached **Figures 1** to **4****:**
According to a first aspect directed to the system, a system is provided that comprises at least one lysis chamber, at least two amplification chambers and at least one luminescence, preferably a fluorescence detection device. The individual lysis chamber may contain a lysing fluid that causes lysing of the cells in a sample to thus release the nucleic acids (DNA or RNA) of a biological sample of interest. The lysing fluid may comprise an acid, e.g. HCI, K acetate or a weak alkali, and a surface active agent.

The amplification chamber may preferably contain a mixture for performing an isothermal method of the present invention. Favourably, the arrangement of the device allows an easy integration of more than one amplification chamber, which provides for a fast multiplex analysis of one and the same sample provided to the at least one lysis chamber.

A biological sample to be analyzed can be provided to the chamber directly after the sample is obtained to guarantee (i) no contamination during transport and (ii) an immediate analysis of the sample. The amplification chamber may further contain exonuclease III in case an "exo probe" for real-time, fluorescence detection is used (cf. Examples below). Further, at least a second amplification chamber may be present. The use of an exonuclease III + probe setting as detailed herein turned out to be particularly efficient and fast: in real time settings, as they will be present in a portable system as disclosed herein for performing a diagnostic reaction using the methods as disclosed herein. The lysed or otherwise pre-treated sample may then be individually transferred to the individual amplification chambers via sterile channels in the device.

In certain preferred embodiments, the magnesium (Mg), for example magnesium acetate, can be added to a lysis buffer or another buffer or a solid agent to be dissolved in a buffer, wherein it is important that the Mg is kept separately from the enzymes driving the amplification reaction so that the reaction can be started on demand by adding Mg in a controllable manner.

When the amplification method of the present invention is performed on a device or system as claimed and disclosed herein, applying a simple rotary motion, or pushing on a button placed on the exterior surface of the device may help the user to add the starting agent, including Mg, and all further necessary reagents to the reaction chamber for analyzing a biological sample of interest. Preferably, no expert knowledge or skills of a trained molecular biologist will be required to start the reaction, which is a significant advantage over the current testing systems in highly-specialized laboratories.

In another embodiments, an internal positive control (IPC) can be contained in the lysis buffer. In another embodiment, the IPC can be provided and the control reaction can be conducted in a separate chamber.

When using a kit of the present invention together with the methods and the device/system of the present invention, the kit will comprise a sterile set to extract a biological sample of interest, preferably in a non-invasive manner, so that the sample can then be transferred by the user/potential patient in the upper chamber of the device/system so that the biological sample can then be contacted with the lysis buffer etc. so that an inactivation of potentially contagious material, a release of genetic material, and an amplification reaction can be achieved/conducted within the device automatically.

The combination of the lysis chamber and piston is arranged such that in the event of the piston moving into the lysis chamber pressure release is possible through venting. The venting means are configured to prevent content of either the lysis chamber or the amplification chamber or both from escaping out of the respective chamber or chambers.

The system is configured to implement an isothermal amplification method such as RPA and SIBA and other methods, preferably isothermal methods. The amplification method (with a standard RPA or SIBA) was usually configured to be carried out in a temperature range between around 25°C and around 47°C. Still, certain practical issues showed that higher temperatures above 37°C may be preferable to obtain a faster reaction kinetics - and thus faster results, the latter being highly favorable for diagnostic applications. Therefore, a completely new design of the biochemical reaction turned out to be of importance, which would work at isothermal temperatures during amplification of around 37°C to about 85°C, preferably of about 39°C to about 60°C, and most preferably from about 40°C to about 55°C. The latter optimum range may be important due to practical reasons, simply in view of the fact that the system was additionally configured to work as a portable handheld device for which regulatory issues have to be taken into consideration to have a device safe in use.

In various embodiments, initial heating may be applied. In certain embodiments, electrical heat may be applied. In other embodiments, the system may additionally, for portable use,

In another embodiments implement continuous heating. There are also embodiments without any external heating. in one or more of the method steps.

The luminescence, preferably the fluorescence detection device may comprise:
- a detection chamber configured to receive the amplification chamber or the contents of the amplification chamber,
- at least one light source,
- at least one optical sensor
- an energy supply
- a wireless data interface and
- a controller.

The controller can be a microcontroller and/or a state machine.

The luminescence, preferably the fluorescence detection device may further comprise heating means that allow heating of an amplification chamber that is inserted in the luminescence, preferably the fluorescence detection device.

The system can either be a point of care (POC) system wherein the luminescence, preferably the fluorescence detection device is arranged at a point of care, for instance in a medical doctor's office. Alternatively, the system may be a personal system wherein the luminescence, preferably the fluorescence detection device is self-contained and mobile, in particular pocketable.

A further aspect of the invention is a set of at least two distinct chambers that can be combined to form a single, fluid tight assembly, wherein a first chamber comprises a first set of chemicals and/or agents, said first chamber being closed prior to use, and wherein a second chamber comprises a second set of chemicals and/or agents that are at least in part distinct from the chemicals and/or agents of the first set, and wherein the first chamber comprises a lid, that can be opened when the first chamber and the second chamber are combined to form a single, fluid tight assembly, in order to allow the contents of the first chamber to enter the second chamber.

Preferably, each chamber is an integral unibody. In an alternative embodiment, the assembly of the first chamber and the second chamber is an integral unibody.

Preferably, the chambers can be connected by means of a fluid tight snap fit connection to thus form a single, fluid tight assembly. As an alternative to a snap fit connection, a screw lock connection similar to a Luer-lock can be provided for tightly connecting the chambers. Another alternative is a press fit connection between the chambers.

The advantage of a fluid tight assembly of at least two chambers is that the assembly can be disposed easily without the risk of contamination because the contents of the assembly is tightly secured within the assembly.

Preferably, the second chamber has transparent walls that allow excitation and detection of luminescence and in particular fluorescence.

An assembly comprising two initially separate chambers and a luminescence, preferably a fluorescence detection device with a receptacle that can receive the assembly for luminescence detection makes it possible to perform a testing method that comprises two consecutive chemical or biochemical method steps - for instance lysis and amplification - and a luminescence testing step in a simple, clean and safe manner that minimizes the risk of contamination and infection while providing easy handling. Alternatively, if no lysis is necessary as DNA or RNA material is directly provided in a rather pure state free of cellular debris or material, the two chambers can be used for parallel amplification reactions for multiplexing purposes, or, in diagnostic settings, valuable controls can be included, or any combination thereof. The luminescence, preferably the fluorescence detection device can be re-used because in use it is not contacted by the sample or any agents or chemicals since these are tightly enclosed in the assembly of chambers. The assembly of chambers and its contents can be safely disposed after use because the contents is reliably kept within the interior of the assembly.

The system 10 for detecting a target analyte comprises a lysis chamber 12, an amplification chamber 14, a piston 18 and a luminescence, preferably a fluorescence detection device 16; see **Figures 1** to **4****.**

A first container 12 is a lysis container that encloses a first chamber comprising a fluid and/or an agent for lysing a sample. The second container 14 is a test container 14 that encloses a second chamber comprising reagents, for instance enzymes for amplifying nucleic acids in a sample that was lysed in the lysis container 12; cf. **Figure 1****.**

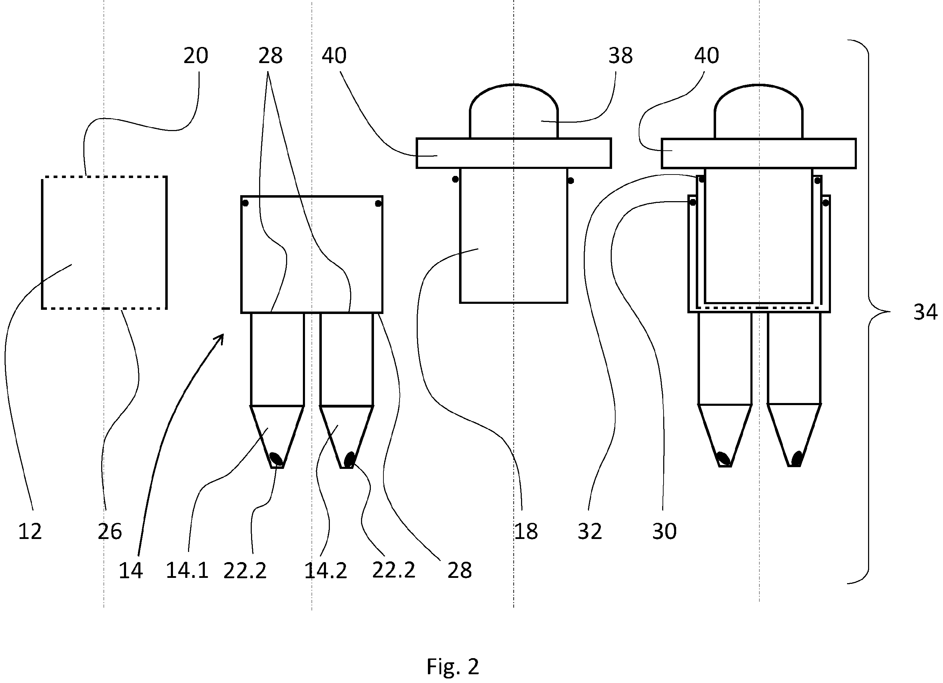

The set of two containers 12 and 14 further comprises means that allow a limited transfer of fluid from the first chamber into the second chamber. In the embodiment shown in **Figures 1** and **2****,** the means for limited transfer of fluid from the first container 12 to the second container 14 comprise a piston 18.

Preferably, two or more amplification or test chambers 14.1 and 14.2 are provided; cf. **Figure 2****.** The test chambers 14.1 and 14.2 can be part of a unitary second container that can be combined with a first e.g. lysis container.

Notably, this configuration of the device is of particular importance for any multimeric assay for nucleic acid amplification, which is presently highly needed in the diagnostic and prognostic practice, but which cannot be realized reliably. In the setting as shown in **Figure 2****,** each of the at least two, at least three, at least four, or at least five, or more, amplification chambers can be pre-equipped with the specific primers and/or probes for amplifying a target sequence of interest, whereas the overall reaction components are the same, as the basic biochemical detection mechanism is the same. More than five parallel reactions can be performed taking into consideration that the system of the present invention, and the relevant reaction chambers, can be subjected to additional nanotizing.

A huge advantage of this design is the fact that potentially contagious material does not have to be obtain from a patient or from an abiotic surface twice, or several times reducing the risk of contamination (of the analyte or sample to be analyzed, and of a person getting in contact with the material). Moreover, the analyte or sample can be analyzed directly in situ and in short time and allows a multiplexing approach to identify a panel of target nucleic acid sequences simultaneously allowing a much better significance and validity of a test. Further, the simultaneous testing can be cost sensitive, as no subsequent test series have to be conducted in case, for example, a first test for a first target sequence is negative, but a patient still shows symptoms of a disease yet to be determined precisely to provide the adequate measures.

The lysis chamber 12 contains a lysing fluid that causes lysing of cells or viruses in a sample to be tested. By way of lysing, nucleic acids such as DNA or RNA are released by way of breaking down the cells or viruses in the sample to be tested. Lysing can be achieved by a lysing fluid that comprises an acid such as hydrochloric acid or a weak alkali. The lysis chamber 12 has a lid 20 so lysis chamber 12 can be opened and a sample to be tested can be entered in the lysis chamber 12. The contents of the lysis chamber 12 is about 100 µl (max 550 µl), but can vary. The lysis chamber composition can also be of such a kind, if a biological sample of interest comprises potentially contagious material, that a rapid inactivation of an infectious/contagious material is achieved.

Lid of the lysis chamber preferably is a membrane 20 that can be pierced by a cotton swab with a sample.

The detection system 10 further comprises two amplification chambers 14.1 and 14.2 that are part of one second container 14.

Each amplification chamber 14.1 and 14.2 comprises a specific mixture of enzymes that are needed for a recombinase polymerase amplification (RPA) of a desired test analyte. Preferably, the mixture is provided in the form of a dry pellets 22.1 and 22.2 that is contained in the amplification chamber 14.

The amplification chambers preferably cuvette-shaped and can be inserted in a receptacle 24 of the luminescence, preferably the fluorescence detection device 16. The receptacle 24 is part of a detection chamber 26 of the luminescence, preferably the fluorescence detection device 16.

The first container 14 is dimensioned to allow inserting the lysis chamber 12 into the first container 14 in a tight and defined manner. An abutment 28 limits the depth of insertion. Once the lysis chamber 12 is fully inserted into the test container 14, the piston 18 can be used to transfer the fluid from the lysis chamber 12 into the amplification chambers 14.1 and 14.2 and allowing the recombinase polymerase amplification to work in each amplification chamber 14.1 and 14.2. To allow the transfer of the contents of the lysis chamber 12 into the amplification chambers 14.1 and 14.2, further thin lids 28 are arranged at the bottom of the lysis chamber 12. The thin lids 28 are dimensioned to break under the fluid pressure caused by the piston 18. Alternatively, the piston 18 and the thin lids 28 can be designed so that tips (not shown) of the piston 18 can pierce the thin lids 28.

The lysis chamber 12, the second container 14 and the piston 18 are configured to engage in a fluid-tight manner when fully inserted. Such fluid tight engagement can be achieved by means of a snap fit connection wherein an annular protrusion 30 of one of the lysis chamber 12 and the second container 14 engages in an annular groove of the respective other part. Likewise, an annular protrusion 32 of one of the piston 18 and the lysis chamber 12 engages in an annular groove of the respective other part. Annular protrusions 30 and 32 act as sealings and may be integrally formed with the rest of the respective chamber or piston.

As an alternative to a snap fit connection, a screw lock connection similar to a Luer-lock can be provided for tightly connecting the lysis chamber 12 and the second container 14. To allow insertion of the lysis chamber 12 into the second container 14 and of the piston 18 into the lysis chamber 12, venting means (not shown) are provided.

Once fully engaged, the second container 14, the lysis chamber 12 and the piston 18 form a tight assembly 34 that can be handled as a single, fluid tight unit.

Walls 36 of the amplification chambers 14.1 and 14.2 are transparent so as to allow light to enter the amplification chambers 14.1 and 14.2 and to exit the amplification chambers 14.2 and 14.2. The transparent walls 36 of the amplification chambers 14.1 and 14.2 make it possible to expose the content of amplification chambers 14. 1 and 14.2 to exiting light that can cause a luminescence. In case, the content of each amplification chamber is luminescent luminescence of the sample in the respective amplification chamber 14.1 or 14.2 can be detected through the transparent walls 36 of the amplification chamber 14.1 or 14.2.

Each pellet 22 containing the mixture of enzymes of four recombinase polymerase amplification preferably comprises a recombinase, a single-stranded DNA-binding protein (SSB) and a strand-displacing polymerase, exonuclease III and in case RNA is to be detected, a reverse transcriptase.

In use, a sample to be tested first is filled into lysis chamber 12. After lysis, the cells in the biological sample to be tested, the entire content of lysis chamber 12 is transferred into the amplification chambers 14.1 and 14.2 by means of the piston 18 so that a recombinase polymerase amplification can occur in the amplification chamber 14.

Once the recombinase polymerase amplification has occurred in the amplification chamber 14.1 or 14.2 - typically between 10 to 15 minutes after filling in the content of the lysis chamber 12 into the amplification chambers 14.1 and 14.2 - the amplification chambers 14.1 and 14.2 - or alternatively only is content - can be entered into the luminescence, preferably the fluorescence detection device 16.

In the illustrated, preferred embodiment, the entire assembly 34 is inserted in the receptacle 24 of the luminescence, preferably the fluorescence detection device 16. For handling of the assembly 34, a grip 38 is provided at a proximal end of the piston 18.

In order to prevent external light, for instance stray light, from entering into the receptacle once the assembly 34 is fully inserted in the receptacle 24, a collar 40 is provided that forms a lid for the receptacle 24.

The luminescence, preferably the fluorescence detection device 16 preferably comprises two detection chambers 42.1 and 14.2 that are configured to receive the amplification chambers 14.1 and 14.2. Within the detection chambers 42.1 and 42.2 or adjacent to the detection chambers 42.1 and 42.2 light sources 44.1 and 44.2 and optical sensors 46.1 and 46.2 are arranged, e.g. one light source and one optical sensor for each detection chamber 42.1 and 42.2. Each light source 44.1 and 44.2 is configured to illuminate the contents of the detection chamber 42.1 or 42.2, respectively, with a light that can cause luminescence in a sample to be tested during and after the sample has undergone recombinase polymerase amplification. Each optical sensor 46.1 and 46.2 is arranged and configured to detect luminescence in the respective detection chamber 42 in case luminescence occurs.

The detections chamber 42.1 and 42.2 are separated by at least one opaque wall 70 that prevents light from one detection chamber 42.1 or 42.2 from entering the respective other detection chamber 42.2 or 42.1.

For powering up the light sources 44.1 and 44.2 and the optical sensors 46.1 and 46.2, an energy supply 48 is provided. The energy supply 48 may comprise a battery, preferably a rechargeable battery. Alternatively or additionally, energy supply 48 may comprise a power interface for connecting the luminescence, preferably the fluorescence detection device 16 to an external power supply. The power interface may be wire-bound or wireless. The energy supply 48 may also comprise solar cells for providing photovoltaic power supply.

The light sources 44.1 and 44.2 and the optical sensors 46.1 and 46.2 are further connected to controller 50 that is configured to control the operation of the light sources 44.1 and 44.2 and the optical sensors 46.1 and 46.2 and to further read out a sensor output signals provided by the optical sensors 46.1 and 46.2. Controller 50 can be a microcontroller or a state machine.

The controller 50 is operatively connected to a wireless data interface 52 that is configured to allow for a data communication between the microcontroller 50 and an external device such as a mobile phone or another device for data communication and data processing. Preferably, the wireless data interface 52 is operatively connected to the controller 50, to the energy supply 48 and to a data memory 54 and is configured to provide for energy harvesting data storage and data communication. In particular, the wireless interface 52 implements means for near field communication (NFC) and comprises a data bus such as a I2C data bus 56 for communication with the controller 50. The wireless data interface 52 preferably is configured to allow bidirectional data communication so as to transmit data generated by the luminescence, preferably by the fluorescence detection device 16 to an external device and to receive the control commands and/or software updates from an external device so that the luminescence, preferably the fluorescence detection device 16 can be controlled and updated by way of an external device.

The wireless data interface 52 may also implement WIFI-communication as an alternative to near field communication. Another alternative is Bluetooth-communication.

Preferably, all electronic components that implement the wireless data interface 52, the controller 50 and the data memory 54 are arranged on a flat printed circuit board 60. The flat printed circuit board 60 can be part of a laminated card wherein the electronic components are arranged between two laminated cover sheets 62. Electric contacts 64 on the surface of one or both cover sheets 60 allow communication and energy transfer between the electronic components and further components such as the light source 44 or the optical sensor 46. Alternatively, in other embodiments, the printed circuit board can be flexible, semi-flexible or a rigid-flexible board. The printed circuit board may be a conventional circuit board or a circuit board manufactured by printing or other additive methods, or combinations thereof.

In addition to the electronic components, the printed circuit board 60 that can be laminated between two cover sheets 62 also comprises at least one antenna 66 for wireless data communication.

Optionally, heating means can be provided. The heating means may comprise an electric heating that is integrated into the luminescence, preferably into the fluorescence detection device 16 and that can heat the assembly 34 and its contents to thus start and/or promote the amplification process. Preferably, the luminescence, preferably the fluorescence detection device 16 is configured to automatically start electric heating one the assembly 34 is inserted into the receptacle 24. The insertion of the assembly 34 can be detected by means of a dedicated sensor, for instance a switch, or by means of the optical sensor 46. In particular, the optical sensor 46 can sense that the assembly is inserted into the receptacle 24 because the collar 40 prevents external light from entering the detection chamber 42 once the assembly is inserted in the receptacle 24. Accordingly, insertion of the assembly 34 into the receptacle 24 causes a drop in light intensity sensed by the optical sensor 46. Such drop in light intensity can be detected and can cause a starting signal for the electric heating.

Alternatively or additionally, the heating means may be provided by chemicals in the amplification chambers 14.1 and 14.2 that undergo an exothermal reaction once a sample is filled in the amplification chambers 14.1 and 14.2. It is also possible that the lysis chamber 12 comprises a first component and the amplification chambers 14.1 and 14.2 comprise a second component wherein the two components can react exothermically. The exothermal reaction begins when the contents of the lysis chamber 12 is transferred into the amplification chambers 14.1 and 14.2.

Alternatively or additionally, the heating means may be provided by chemicals that undergo an exothermal reaction once a sample is introduced into the receptacle. These chemicals are arranged in proximity of the amplification chambers 14.1 and 14.2, in a separate sealed arrangement that contains the heating chemicals. Upon a trigger, the exothermic chemical reaction is started. This can be suitable when the system is used, for example, as a portable diagnostic tool.

Another alternative that allows heating of the luminescence, preferably the fluorescence detection device is proving a dark colour, for instance black, on the outside of the fluorescence detection device. Thus, for example, a fluorescence detection device together with the amplification chamber can be solar heated. Preferably, temperature control means are provided that indicate potential overheating of the fluorescence detection device together with the amplification chamber. The temperature control means may comprise an ink or paint that changes its colour in case a predefined temperature is exceeded. Accordingly, an indicator that changes its colour at a certain temperature and that is applied to the outside of the fluorescence detection device may be a temperature control means.

The detection chambers 42.1 and 42.2 are arranged in a detection chamber housing 72 that has outer dimension smaller than 10 cm by 10 cm by 4 cm. Preferably the volume of the entire luminescence, preferably of the fluorescence detection device 16 is smaller than 200 cm², and even more preferred smaller than 100 cm². In a preferred embodiment, the longest outer dimension is a least twice as long as the shortest outer dimension.

In yet a further aspect of the present invention, there is provided a kit for performing a method as disclosed herein, preferably on a system as disclosed herein, wherein the kit comprises (i) at least one recombinase, at least one single-strand binding protein, at least one DNA polymerase, and optionally: at least one reverse transcriptase and/or at least one energy regeneration enzyme, together with suitable reaction components, wherein (ii) at least two target sequence specific primers; (iii) suitable reaction components, including at least one co-factor for at least one enzyme, dNTPs or a mixture of dNTPs and ddNTPs, at least one buffer system, at least one reducing agent, and adenosine triphosphate; (iv) at least one sterile set for extracting a biological probe to be analyzed and comprising at least one component configured to be applied to a system as disclosed herein, preferably into the lysis chamber (12) of the system; (v) optionally: at least one probe and optionally at least one enzyme activating the probe; and (vi) optionally: at least one inhibitory agent blocking the activity of the at least one recombinase and/or optionally: at least one recombinase-assisting factor; and (vii) optionally: wherein the kit further comprises a second part comprising (i) to (v), wherein the second part comprises a predetermined second target sequence acting as positive control and at least one, preferably at least two primers specific for the second target sequence, and preferably a second probe. Optionally, the kit can further comprise instructions for use.

In yet a further aspect, there is provided a set of kits, wherein the sum of these kits makes up a panel for diagnosis, which is easily customizable. All ingredients needed for each individual amplification chamber can be provided as individual kit of the set of kits. The individual part can be provided as pellet, i.e., for example, lyophilized, and/or as partly liquid part. In certain embodiments, the liquid may be provided separately, or as separate compartment, so that the activation of the biochemical mechanism is initiated only after a sample is provided to the device, particularly to the at least one lysis chamber.

Preferably, all components of a device or kit of the present invention are provided in a sterile form.

In a laboratory, the skilled molecular biologist or trained personnel can easily conduct the methods as disclosed herein, design primers and/or probes for target nucleic acids to be amplified, and suitable reaction conditions can be easily varied, if desired. Of course, it is a much bigger challenge to perform the method in a non-laboratory environment. Particularly in the latter situation, it may be favourable to provide a kit comprising all necessary components in ready-made sterile vials or tubes, so that the (bio-)chemical agents and components can easily be re-activated (for enzymes, for example, after freeze-drying) and the reaction can be started in a convenient manner.

In the embodiment, where a system of the present invention is provided to perform a diagnostic method of the invention in a portable device, it may be highly suitable that the kit comprises optimized primers to detect the genetic material of an agent, usually an infectious agent, as target sequence in a highly specific manner. Further, it will be useful to provide a sterile set, including gloves, to extract a biological sample in a preferably non-invasive manner.

An infectious agent as used herein may include an infectious agent of all taxa, including viruses. The infectious agents thus may include a virus, a bacterium, but also any kind of eukaryotic organism, including a yeast, fungus, nematode etc. The term infectious agent may also include an element of an infectious agent, e.g., an element or plasmid encoding an antimicrobial resistance (AMR) element mediating antibiotic resistance representing an escalating threat. In a preferred embodiment, the sterile extraction part of the kit will be configured so that the sample retrieval device will be compatible with at least one chamber, preferably the lysis chamber, of the system of the invention so that the sample retrieval device including the biological sample to be analyzed can be easily applied to the respective chamber of the device.

Is detailed for the methods of the present invention, these methods have been designed in a way that less enzymes and chemical agents are needed than for earlier isothermal amplification methods like SIBA or RPA. This simplicity and reduced complexity of the method also has advantages, as the various parts of the kit needed can be reduced in case the relevant biochemical reaction on the sample can be conducted in a more stable and easier manner.

In yet another aspect, there may be provided the use of at least one thermostable enzyme of the methods of the present invention, or a use of at least one nucleic acid sequence encoding the same, optionally for use in combination with a kit as defined above, for performing an isothermal method of nucleic acid amplification of at least one target sequence. The use of at least one thermostable enzyme as disclosed herein can be of particular interest in a variety of isothermal nucleic acid amplification assays performed under laboratory as well as under point-of-care and/or even under home environment conditions.

The present invention will now be further illustrated with reference to the following nonlimiting Examples.

### Examples

### Example 1: Establishing an RT-RPA for SARS-CoV-2

Initially, a real-time reverse transcription RPA (RT-RPA) targeting the RNA-dependent RNA polymerase (RdRP), envelope protein (E) and nucleocapsid (N) genes of SARS-CoV-2 was established to determine the limit of detection, cross reactivity and clinical performance in comparison to real-time RT-PCR (Abd EI Wahed et al., Suitcase lab for rapid detection of SARS-CoV-2 based on recombinase polymerase amplification assay, J. Clin. Virol., under review).

For assay validation, molecular RNA standards based on the RdRP -, E- and N-gene (Accession number: NC_045512, nucleotides: 14977-15975, 26112-26479 and 28280-29536, respectively) were synthesized by GenExpress (Berlin, Germany). Oligonucleotides for the RdRP and E genes were updated from our previous design for SARS-CoV-1 and MERS CoV (unpublished data), whereas the N gene amplicon was modified from a previously published article (Behrmann, O. et al. Rapid detection of SARS-CoV-2 by low volume real-time single tube reverse transcription recombinase polymerase amplification using an exo probe with an internally linked quencher (exo-IQ). Clin Chem, doi:10.1093/clinchem/hvaa116 (2020)). **(Table 1).** All oligonucleotides were synthesized by TIB MOLBIOL GmbH (Berlin, Germany).

**Table 1. RT-RPA assay oligonucleotides:**

| **Gene** | **Oligonucl eotide** | **Sequence 5'-3'** |
|---|---|---|
| RdRP | Forward | TATGCCATTAGTGCAAAGAATAGAGCTCGCAC (SEQ ID NO: x) |
| | Reverse | CAACCACCATAGAATTTGCTTGTTCCAATTAC |
| | Exo-Probe | TCCTCTAGTGGCGGCTATTGATTTCAATAAbTXfTTTGATGA AACTGTCTATTG-PH |
| E | Forward | GAAGAGACAGGTACGTTAATAGTTAATAGCGTA |
| | Reverse | AAAAAGAAGGTTTT ACAAGACTCACGTT AACsA |
| | Exo-Probe | ATCGAAGCGCAGTAAGGATGGCTAGbTXfTAACTAGCAAGA ATAC-PH |
| N | Forward | CCTCTTCTCGTTCCTCA TCACGT AGTCGCAAC |
| | Reverse | AGTGACAGTTTGGCCTTGTTGTTGTTGGCCTT |
| | Exo-Probe | TAGAATGGCTGGCAATGGCGGTGATGCTGCbTXfTTGCTTT GCTGCTGCTT-PH |

BHQ1-dt (bT), Tetrahydrofuran (X), Fam-dT (fT), phosphothioate backbone (s) and PH: 3' phosphate to block elongation.

### Example 2: RT-RPA reaction conditions

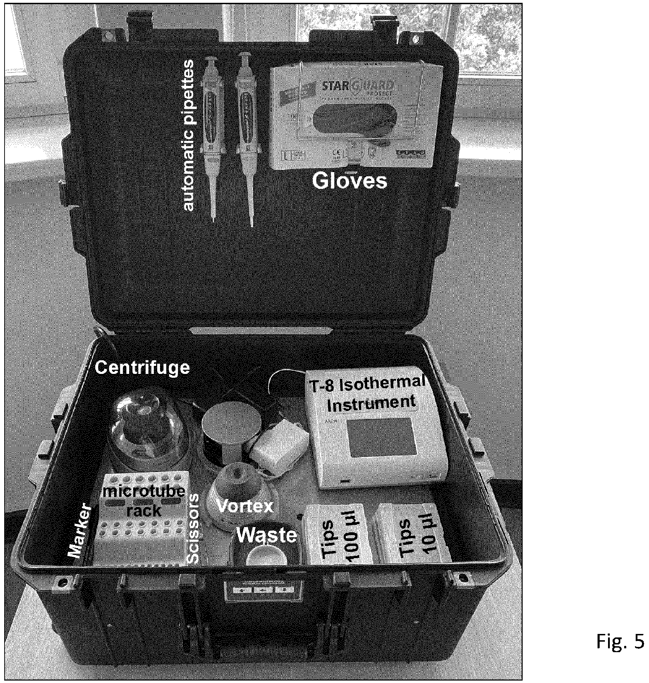

For RT-RPA, a TwistAmp exo kit (TwistDx, Cambridge, UK) was used in combination with lyophilized RevertAid reverse transcriptase (Life Technologies, Darmstadt, Germany). Per reaction, 29.5 µl rehydration buffer, 2.5 µl of RevertAid reverse transcriptase (200 U/µl), 9.7 µl H₂O, 2.1 µl of forward primer (10 pmol/µl), 2.1 µl of reverse primer (20 pmol/µl), 0.6 µl of exo-probe (10 pmol/µl), 2.5 µl of 280 mM magnesium acetate and 1 µl of the template were added into the lid of the reaction tube containing the freeze-dried pellet. The tube was closed, centrifuged, mixed, centrifuged and placed immediately into the T8 (Axxin, Fairfield, Australia) isothermal fluorescence reader. The reaction was incubated at 42°C for 15 minutes. A mixing step was conducted after 230 seconds for the RdRP and E RT-RPA assays and after 320 seconds for the N RT-RPA assay. All procedures were performed in a mobile suitcase laboratory **(****Figure 5****,** Bodo Koennecke, Berlin, Germany). The threshold time (TT) was calculated as the starting point of the amplification curve above the threshold of the negative control (water as template) in the 1^{st} derivative analysis in the T8 Desktop software (Axxin, Fairfield, Australia).

### Example 3: RT-RPA analytical sensitivity and specificity

Serial dilutions of the molecular standards (10⁶-10⁰ RNA molecules/per reaction) were screened in the respective RT-RPA assays. To determine the minimal number of RNA molecules reaction detected in 95% of cases, a probit regression analysis of five data sets from RPA reactions with the complete standard dilution range was performed using STATISTICA software (StatSoft, Hamburg, Germany), and the graph was created by GraphPad PRISM version 6.07 software (GraphPad Software Inc., San Diego, California). The analytical specificity of the RT-RPA-assays was tested with viral genomes of the viruses listed in **Table 2.** The nucleic acids extracts were provided by Charite Medical University (Berlin, Germany), Robert Koch Institute (Berlin, Germany), Landesgesundheitsamt Niedersachsen (Hannover, Germany), Quality Control for Molecular Diagnostics (Glasgow, Scotland) and the Friedrich-Loeffler-Institute (Greifswald-Insel Riems, Germany).

The three RT-RPA-assays were validated with leftover RNA extracts from suspected COVID19 cases diagnosed at the Leipzig University Hospital by real-time RT-PCR (E based assay as screening test and RdRP as confirmatory test). A total of 18 positive and 18 negative samples (blinded) were tested. Diagnostic sensitivity and specificity, positive predictive value and negative predictive value for the RT-RPA assays were calculated using standard formulas against the real-time RT-PCR as reference test (Altman, D. G. & Bland, J. M.: Diagnostic tests 2: Predictive values. BMJ 309, 102, doi:10.1136/bmj.309.6947.102 (1994)).

The RNA extracts from patient materials were then tested with a real-time PCR assay combining commercial SARS-CoV-2-E oligonucleotides (TIB MOLBIOL GmbH, Berlin, Germany) and Superscript III Platinum One-Step qRT-PCR kits (Life Technologies, Darmstadt, Germany) using the following temperature profile: 50°C for 15 minutes, 95°C for 2 minutes followed by 40 cycles of 95°C for 15 seconds and 60°C for 30 seconds on a Agilent Technologies Stratagene Mx 3000p Real-Time PCR system.

### Analytical Sensitivity and Specificity

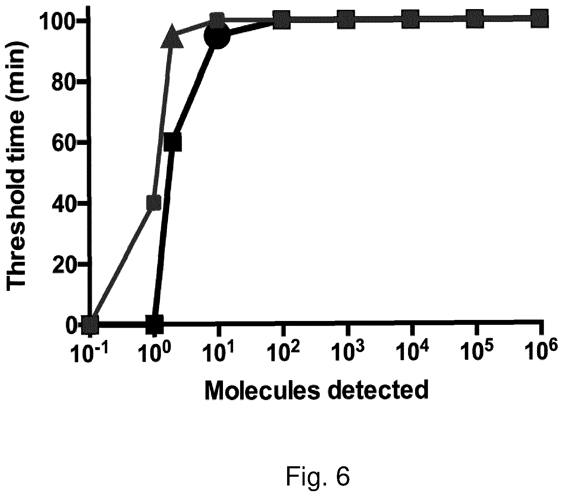

To determine the analytical sensitivity of the RdRP, E and N RT-RPA assays as such, each assay was tested with a serial dilution of 10⁶-10⁰ RNA molecules/reaction of the respective molecular RNA standard in five replicates. Molecular RNA standards with the concentration from 10⁶ to 10² molecules/reaction were detected in all five RPA runs of the three RT-RPA-assays. The E and N RT-RPA identified 100 RNA molecules in 3/5 RT-RPA runs, while in RdRP-RT-RPA, all 5 runs were positive. Only the RdRP RT-RPA amplified one RNA copy in 2/5 RT-RPA runs. With this data set, probit regression analysis was performed and revealed a 95% detection limit of 2 RNA molecules for the RdRP RT-RPA assay, and 15 RNA molecules for E and N RT-RPA assays **(****Figure 6****).**

RdRP- and E- RT-RPA assays were thus able to amplify genomic RNA of both SARS-CoV-1 and -2, whereas the N- RT-RPA assay recognizes SARS-CoV-2 RNA only. None of the assays showed cross reactivity to nucleic acid extracts from other respiratory viruses listed in **Table 2.**

**Table 2: Viral genomes analyzed for the cross reactivity by the three SARS-CoV-2 RT-RPA assays.**

| **Viral nucleic acid** | **RdRP** | **E** | **N** |
|---|---|---|---|
| SARS-CoV-2 | + | + | + |
| SARS-CoV-1 | + | + | - |
| Coronavirus 229E | - | - | - |
| Coronavirus NL63 | - | - | - |
| Coronavirus OC43 | - | - | - |
| MERS-Coronavirus | - | - | - |
| Influenza A (H1N1 pdm09) | - | - | - |
| Influenza A (H3N2) | - | - | - |
| Influenza A (H5N1) | - | - | - |
| Influenza A (H1N1 H275Y) | - | - | - |
| Influenza B (Victoria) | - | - | - |
| Influenza B (Yamagata) | - | - | - |
| Parainfluenza virus 1 (patient isolate) | - | - | - |
| Parainfluenza virus 2 (patient isolate) | - | - | - |
| Parainfluenza virus 3 (patient isolate) | - | - | - |
| Parainfluenza virus 4 (patient isolate) | - | - | - |
| Respiratory syncytial virus A and B | - | - | - |
| Human rhinovirus A 16 | - | - | - |
| Human rhinovirus B 5 | - | - | - |
| Human metapneumovirus A1 | - | - | - |
| Human metapneumovirus B2 | - | - | - |
| Adenovirus type 1 | - | - | - |
| Adenovirus type 4 | - | - | - |
| Adenovirus type 34 | - | - | - |
| A/Anhui/1/13 (H7N9) | - | - | - |
| A/ Chicken /Germany/79 "Taucha" (H7N7) | - | - | - |
| A/Chicken/Brescia/19/02 (H7N7) | - | - | - |
| A/Cygnus olor/Germany/R1377/07 (H5N1) | - | - | - |
| Newcastle disease virus clone 30 | - | - | - |
| Infectious laryngotracheitis virus U76 | - | - | - |
| Infectious bronchitis M41 | - | - | - |

### Example 4: Further tests on clinical samples

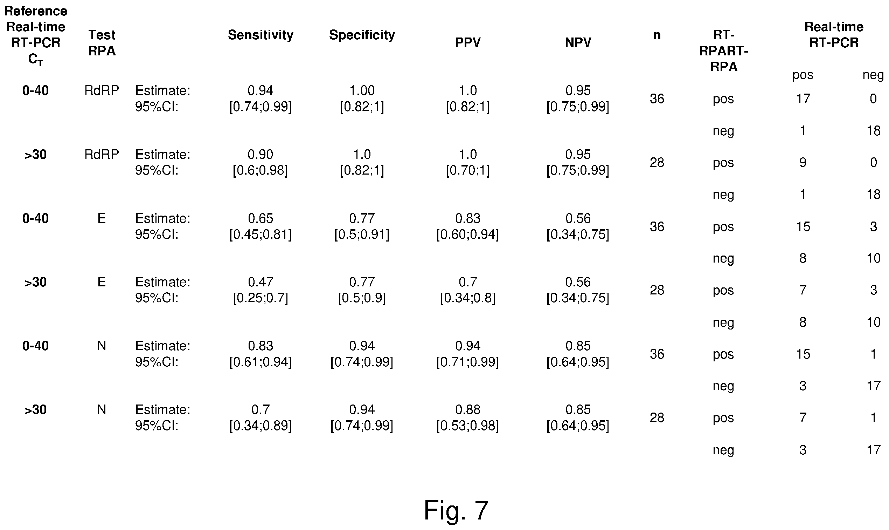

All extracted RNA samples which had been stored at -80°C were first tested by the three RT-RPA assays and retested by real-time RT-PCR to confirm previous results. The RT-RPA assay targeting the RdRP gene produced the best clinical performance (Figure 7). All RT-RPA assays detected samples with a *C_{T}*>30 at a maximum of 12 minutes (data not shown here, cf. Abd EI Wahed et al., *supra).* To identify a highly sensitive and specific SARS-CoV-2 RPA assay, three RT-RPA assays targeting the RdRP, E and N genes of the SARS-CoV-2 genome were evaluated. The RdRP RT-RPA assay produced sensitivity and specificity similar to a real-time RT-PCR within less than 15 minutes. The speed and the clinical accuracy of the RT-RPA assay make it an ideal candidate molecular assay at point-of-need.

It could thus be demonstrated that an isothermal nucleic acid amplification method can provide highly reliable results in the diagnosis of a SARS-CoV-2 infection, where real-time RT-PCR is currently the method of choice for molecular laboratory screening of samples from suspected COVID-19 patients. In general, state and commercial diagnostic laboratories in developed countries have all the infrastructure for real-time RT-PCR. In emerging nations, usually only reference laboratories are adequately equipped to perform real-time RT-PCR assays with high reliability though and alternative testing formats are thus urgently needed.

Even though the classical RPA assays may be perfectly suitable for the diagnosis of SARS-CoV-2 in a laboratory environment when performed by trained personnel, we further tried to optimize the method so that it will be suitable for high-throughput reliable testing in a non-laboratory environment when conducted by any interested individual, still guaranteeing that the results are fast and reliable.

### Example 5: SARS-CoV-2 specific optimizations

First of all, certain improvements had to be introduced during the development of RPA assays specific for SARS-CoV-2. After several rounds of testing, firstly the best assay sensitivity was achieved with using double concentrations of the reverse primer. Coronaviruses have a positive sense single stranded RNA genome and more reverse primer produces more cDNA for the following amplification step. Second, during the oligonucleotide design any sequences pairing to the human genome were strictly excluded in order to avoid problems as reported for some real-time RT-PCR assays. Third, to avoid primer dimer and non-specific amplification, phosphothioate nucleotides were introduced in selected RPA primers.

In addition to the amplification and detection, the virus inactivation and extraction are usually crucial steps in the diagnosis of SARS-CoV-2 in order to assure the safety of the healthcare worker or laboratory technician, sample inactivation is usually recommended in a BSL-2 cabinet and under field condition in a mobile Glove box. Several rapid and simple extraction procedures were developed for coronavirus extraction (Fomsgaard & Rosenstierne, March 2020. Euro Surveill 25, doi:10.2807/1560-7917.ES.2020.25.14.2000398).

Still, for a test in a home environment, we speculated that it will be of less importance to assure virus inactivation. It should be much more important to guarantee an easy way of extracting the sample potentially comprising detectable virus genetic material and to have an even increased stability and simplicity, and what is more: to obtain a testing result even faster. Therefore, new options were tested to design a new isothermal nucleic acid amplification method going beyond the RPA SARS-CoV-2 test, which as such was demonstrated to be perfectly suitable for RNA virus detection.

### Example 6: Analysis and test of thermostable biocatalysts useful in isothermal nucleic acid amplification assays

To establish an isothermal reaction that would be robust enough to be performed by a layman in the specific handheld diagnostic device as developed earlier, it was speculated that one could choose a completely different set of enzymes for performing the isothermal amplification reaction. The driving factor behind these thoughts was the fact that the presently available diagnostic tests for SARS-CoV-2 are still time consuming. Increasing the testing-to-result time is thus of utmost importance to decrease time in quarantine and the risk of a viral spread.

Therefore, several rounds of *in silico* search were performed to identify thermostable enzymes having an enzymatic function allowing the performance of a reliable and fast isothermal amplification reaction. The thermostable nature of the enzymes has two major advantages in practice: the enzymes will not easily degrade before the test is performed and the reaction time can be increased as the enzymes will have optimum activity at temperatures above standard body temperature (∼ 37°C), for which most enzymes are optimized for by nature, so that the amplification of a target DNA or RNA sequence - and in turn a diagnostic result - can be available much quicker.

Searches for thermostable phage enzymes were rather cumbersome, as the relevant genomes are presently only poorly annotated. In the end, a complete set of enzymes having a functionality suitable for an isothermal amplification reaction and further having an optimum temperature above 37°C, and certain enzymes even having an optimum temperature between above 37°C and about 55°C, or even above, (meaning: the optimum temperature for the handheld diagnostic device) were identified.

These enzymes are predominantly derived from bacteria and/or archaea known to be thermostable. Further, in case a reverse transcriptase is needed for RNA targets, several commercially available thermostable enzymes are on the market, and one additional new virus-derived reverse transcriptase was identified.

The enzymes newly identified are summarized in the following **Table 3:**

| Organism | Function | SEQ ID NO: |
|---|---|---|
| Q1 IWF2/ *Deinococcus geothermalis* | Recombinase | 1 |
| Q1IY76/*Deinococcus geothermalis* | RecR-like | 2 |
| Q1J027/ *Deinococcus geothermalis* | RecO-like | 3 |
| Q1J1N6/ *Deinococcus geothermalis* | Single-strand binding/stabilizing protein | 4 |
| AAC37139.1/ Geobacillus stearothermophilus | Polymerase | 5 |
| O40444/HIV-1 O typ | Reverse Transcriptase | 6 |
| Q1J193/ *Deinococcus geothermalis* | Poly-P Kinase | 7 |
| A0A075WCY2/ *Archaeoglobus fulgidus* | Exonuclease III (Exo-III) | 8 |

The *Deinococcus geothermalis* enzymes and the further enzymes detailed in Table 4 above were found to have an optimum activity between 40°C and 60°C, or even above, which clearly shows that these enzymes are thermostable.

Further, the recombinant production and purification of the enzymes has certain advantages (details not shown here). For the final assay, this means that these enzymes can be produced affinity tag-free in sufficient amounts and in sufficient purity without the need to cleave off the affinity tag, or to use an affinity tagged

Finally, the enzymes in view of their functionality and assisted by their thermostable nature, are suited to perform an isothermal amplification reaction even above the 42°C standard of a "classical" RPA reaction, and, therefore, is perfectly configured to perform better on the handheld diagnostic device as recently developed. In first experiments, a positive result could be detected via the cognate fluorescence channel already below 10 minutes. This taken together with the fact that a reliable positive control standard is simultaneously conducted further increasing the safety and reliability, paves the way for a new generation of diagnostics for SARS-CoV-2 and other infectious diseases.

Initially, it could be shown that - already before the amplification assay and important for the non-laboratory use and the difficulties associated with shipping, storage, etc. -the increased stability of the thermostable biocatalysts allows short-term storage before lyophilization at 4°C. Hence, the addition of supplements (e.g. glycerol) negatively interfering with the lyophilization process is avoided. Further, this guarantees a reliable marketing via pharmacies and other retail trade channels when delivering the device including the biochemical ingredients to non-laboratory practice skilled practioners.

Turning now to the methodology, we observed significant advantages over classical isothermal reactions, like RPA, as the nature of the thermostable enzymes allows a less stringent temperature control, but simultaneously allows to decrease the reaction time whilst showing sensitivity and sensibility comparable to the RPA assays for SARS-CoV-2 as detailed below. These factors, of course, are of outstanding importance when providing a kit for performing a diagnostic isothermal nucleic acid amplification reaction where fast test results -in a laboratory as well as a home environment - are of utmost importance.

Further work is underway to additionally, optimize the new assay using the thermostable enzymes also for laboratory settings, independent of the handheld device, as the assay as such performs better (and cheaper) in certain aspects than standard isothermal nucleic acid amplification techniques.

It is expected that the enzyme mix using thermostable enzymes will provide a higher stability of the reaction mix amenable to long term cold chain independent storage, and use at ambient temperatures in the range of about 30 to about 40°C.

### Example 7: Design of a mimic amplicon

For constructing a mimic amplicon for test purposes to establish a new reaction on the specific portable diagnostic device whilst using the new thermostable enzymes, the probe sequence of the SARS CoV 2-RdRP assay detailed above was scrambled twice until it generated no more hits when tested by the BLAST algorithm. The flanking primers (SEQ ID NOs: 18 (forward) and 19 (reverse), respectively) of the SARS CoV 2- RdRP were placed up and downstream of the probe sequence (SEQ ID NO: 20) with a 5 nt spacer interspersed on each side of the probe sequence.

The above mimic amplicon has been used as valuable internal positive control to test the efficiency of the amplification reactions using Twist RPA assays and the new experiments with optimized enzymes and reaction conditions and turned out to be highly specific and reliable. Moreover, the mimic amplicon serves a valuable inhibition control meaning that the amplicon serves as process or amplification control added from the beginning to check whether the overall amplification parameters and perform well. Particularly for diagnostic applications, a control as the mimic amplicon just described is obviously of utmost importance.

## Claims

1. An isothermal method of nucleic acid amplification of at least two target sequences, preferably in a biological sample, the method comprising the steps of:
(a) providing
(i) at least one recombinase, at least one single-strand binding protein, at least one, and preferably at least two target sequence specific primers, at least one DNA polymerase, and optionally: providing at least one reverse transcriptase and/or at least one energy regeneration enzyme, together with suitable reaction components, wherein
(ii) either at least one co-factor essential for the activity of at least one of the enzymes is not provided; or wherein at least one inhibitory agent blocking the activity of the at least one recombinase is provided;
(b) providing at least one biological sample to be analyzed for the presence of the at least one target sequence and optionally providing a preferably sterile extraction kit to obtain the biological sample;
(c) optionally: providing at least one probe and optionally providing at least one enzyme activating the probe so that a detectable signal is generated;
(d) adding a starting reagent to initiate the amplification reaction; and
(e) obtaining the at least one amplified target sequence and/or obtaining at least one detectable signal each signal being indicative of a successful amplification of the respective target sequence amplified.

2. The method of claim 1, wherein at least three, at least four, at least five, at least six, or more than six different target sequences are amplified, preferably in the same sample used.

3. The method of claim 1 or 2, wherein the target sequence is individually selected from the group consisting of a target sequence of an infectious agent, including a target sequence of a virus being selected from Ebola virus, Sudan virus, Marburg virus, Bundibugyo virus, Monkeypox virus, Yellow Fever virus, Zika virus, Japanese Encephalitis virus, Rift Valley Fever virus, Dengue virus, Chikungunya virus, Rabies virus, Influenza virus A or B, including H5N1 and H7N9, Respiratory Syncytial virus, Adenovirus, including Adenovirus 1, 4. 7, Paraifluenzavirus 3, MERS CoV, SARS-CoV-2, Parvovirus B19, West-Nile virus, Herpes simplex virus, Varizella Zoster virus, Norovirus, Bovine Coronavirus, Foot and Mouth Disease virus, Lumpy Skin Disease virus, or including a target sequence of a bacterium, including *Staphylococcus* aureus, including an antimicrobial resistance gene, or including a target sequence of *Mycobacterium ulcerans, Treponema pallidum, Leishmania donovani, Mycobacterium leprae, Fransciella tularensis, Bacillus anthracis, including pacA*/*capC, Clostridum difficile, inlcuding tcdB*/*tcdA, Pan-Ricketsia, Salmonella typhi or paratyphi, Plasmodium falciparum, Mycobacterium avium subs. Paratuberculosis,* a Cytomegalovirus, or an enterovirus.

4. The method of any of the preceding claims, wherein the at least at least two target sequences are provided as part of a systematic panel, wherein the panel is selected from the group consisting of an acute infectious disease panel, a meningoencephalitis panel, a severe respiratory disease panel, an arbovirus panel, an antibiotic resistance gene panel, a country-specific travel returnee panel, a panel specific for a screening during pregnancy, a panel specific for testing individuals before planned or acute hospitalization, a panel for screening for screening for infectious pediatric diseases, including pediatric diseases associated with an erythema, and/or a panel for testing livestock animals.

5. The method of any of the preceding claims, wherein no additional crowding agent is provided and/or wherein at least one recombinase-assisting enzyme or factor is additionally provided, preferably before step (d).

6. The method of any of the preceding claims, wherein at least one energy regeneration enzyme system is provided and/or wherein at least one forward and at least one reverse primer are provided as target sequence specific primers, wherein at least one of these primers, preferably the reverse primer, is provided in a higher amount or concentration in relation to the other primer.

7. The method of any of the preceding claims, wherein at least one probe and/or label is provided, wherein the at least one probe and/or the at least one label comprise a directly or indirectly detectable moiety.

8. The method of any of the preceding claims, wherein the at least one enzyme comprises at least one tag and/or label.

9. The method of any of the preceding claims, wherein the target sequence is a single stranded or double stranded DNA and/or RNA nucleic acid sequence.

10. The method of any of the preceding claims, wherein the temperature during the amplification reaction is about 37°C to about 85°C, preferably of about 39°C to about 60°C, and most preferably from about 40°C to about 55°C.

11. A system for detecting at least two target sequences in a biological sample or in a sample obtained from a surface, preferably a portable system, said system comprising:
at least one lysis chamber (12), at least two amplification chambers (14.1 and 14.2) that are part of one second container (14) and at least one luminescence, preferably at least one fluorescence detection device (16), wherein the lysis chamber (12) contains a lysing fluid, each of the at least two amplification chambers (14.1 and 14.2) contains a mixture that comprises the set of enzymes and the suitable reaction components as defined in claims 1 to 10 for amplifying at least two target nucleic acid sequences, and the luminescence, preferably the fluorescence detection device (16) comprises a detection chamber (42) configured to receive the amplification chamber (14) or the contents of the amplification chamber, a light source (44), an optical sensor (46), an energy supply means (48), a wireless data interface (52) and a controller (50), wherein the system is configured to perform a method according to any one of claims 1 to 10.

12. The system of claim 11, wherein the system is configured so that it can be combined to form a single, fluid tight assembly (34), wherein the at least one first lysis chamber (12) comprises a first set of chemicals and/or agents, said first chamber being closed prior to use, preferably wherein the first set of chemicals and/or agents allows the accessibility of the at least one target nucleic acid sequence to be amplified and/or the inactivation of potentially contagious material in a sample, and wherein a second amplification chamber (14.1 or 14.2) of the at least two amplification chambers (14) each comprises a second set of chemicals and/or agents that are at least in part distinct from the chemicals and/or agents of the first set and being at least distinct from the chemicals and/or agents of the at least one further second amplification chamber set in that parts decisive for target gene specificity, and wherein the at least one first lysis chamber comprises a lid (26), that can be opened when the at least one first lysis chamber (12) and one of the at least two second chambers (14.1 or 14.2) are combined to form a single, fluid tight assembly (34), in order to allow the contents of the at least one first lysis chamber (12) to individually enter each of the at least two second chambers (14).

13. The system of claim 11 or 12, wherein the system additionally comprises a luminescence, preferably a fluorescence detection device (16), the luminescence, preferably the fluorescence detection device (16) comprising:
- a detection chamber (42) configured to receive an amplification chamber or the contents of the amplification chamber,
- a light source (44) configured to excite luminescence and in particular fluorescence,
- an optical sensor (46) configured to capture luminescence and in particular fluorescence,
- energy supply means (48),
- a wireless data interface (52) and
- a controller (50).

14. A kit for performing a method of any one of claims 1 to 10, wherein the kit comprises
(i) at least one recombinase, at least one single-strand binding protein, at least one DNA polymerase, and optionally: at least one reverse transcriptase and/or at least one energy regeneration enzyme, together with suitable reaction components, wherein
(ii) at least at least two target sequence specific primers;
(iii) suitable reaction components, including at least one co-factor for at least one enzyme, dNTPs or a mixture of dNTPs and ddNTPs, at least one buffer system, at least one reducing agent, and adenosine triphosphate;
(iv) at least one sterile set for extracting a biological probe to be analyzed and comprising at least one component configured to be applied to a system as defined in claim 11, preferably into the lysis chamber (12) of the system;
(v) optionally: at least one probe and optionally at least one enzyme activating the probe; and
(vi) optionally: at least one inhibitory agent blocking the activity of the at least one recombinase and/or optionally: at least one recombinase-assisting factor; and
(vii) optionally: wherein the kit further comprises a second part comprising (i) to (v), wherein the second part comprises a predetermined second target sequence acting as positive control and at least one, preferably at least two primers specific for the second target sequence, and preferably a second probe.

15. A use of at least one enzyme as defined in claim 7, or a use of at least one nucleic acid sequence encoding the same, optionally for use in combination with a kit as defined in claim 14, for performing an isothermal method of nucleic acid amplification of at least one target sequence as defined in any one of claims 1 to 10.
